# EUROPEAN PATENT SPECIFICATION

(11) **EP 4 017 998 B1**
(45) Date of publication and mention of the grant of the patent: **06.11.2024**
(21) Application number: 20855794.2
(22) Date of filing: 20.08.2020
(51) Int. Cl.: C12Q 1/6809, C12Q 1/6811, C12Q 1/682, C12Q 1/6827, C12Q 1/6858

(54) **MULTI-SITE ENRICHMENT OF DELETIONS IN DNA MICROSATELLITES**
MEHRSTELLENANREICHERUNG VON DELETIONEN IN DNA-MIKROSATELLITEN
ENRICHISSEMENT MULTI-SITE DE DÉLÉTIONS DANS DES MICROSATELLITES D'ADN

(30) Priority: 21.08.2019 US 201962889960 P
(43) Date of publication of application: 29.06.2022
(73) Proprietor: Dana-Farber Cancer Institute, Inc., Boston, MA 02215 (US)
(72) Inventor: MAKRIGIORGOS, Gerassimos, Chestnut Hill, MA 02467 (US)
(74) Representative: Elkington and Fife LLP
(86) International application number: PCT/US2020/047098
(87) International publication number: WO 2021/034999

(56) References cited:
- WO-A1-2018/111835
- US-A1- 2018 187 242
- LADAS IOANNIS, YU FANGYAN, LEONG KA WAI, FITARELLI-KIEHL MARIANA, SONG CHEN, ASHTAPUTRE RAVINA, KULKE MATTHEW, MAMON HARVEY, MAKRI: "Enhanced detection of microsatellite instability using pre-PCR elimination of wild- type DNA homo-polymers in tissue and liquid biopsies", NUCLEIC ACIDS RESEARCH, vol. 46, no. 12, 6 July 2018 (2018-07-06), pages 1 - 8, XP055794902
- OWCZARZY ET AL.: "Predicting Stability of DNA Duplexes in Solutions Containing Magnesium and Monovalent Cations", BIOCHEMISTRY, vol. 47, no. 19, 13 May 2008 (2008-05-13), pages 5336 - 5353, XP055040115, DOI: 10.1021/bi702363u

## Description

### GOVERNMENT SUPPORT

This invention was made with government support under grant numbers R33 CA217652 and R01 CA221874 awarded by The National Institutes of Health. The government has certain rights in the invention.

### BACKGROUND OF THE INVENTION

A commonly encountered situation in genetic analysis entails the need to identify a low percent of variant DNA sequences (`minority alleles') in the presence of a large excess of non-variant sequences (`majority alleles'). Methods to determine the original abundance of mutant alleles of one or more barcoded target sequences following mutation enrichment and sequencing are described in WO 2018/111835A1.

The following are examples of situations in which minority alleles need to be identified. (a) identification and sequencing of a few mutated alleles in the presence of a large excess of normal (wild-type) alleles, a commonly encountered situation in cancer. (b) identification of a few methylated alleles in the presence of a large excess of unmethylated alleles (or: vice versa) in epigenetic analysis. (c) identification and genotyping of a few fetal DNA sequences circulating in the maternal blood where a large excess of maternal DNA sequences are also present. (d) identification of tumor-circulating DNA in blood or in urine of cancer patients (or abnormal DNA in people suspected of having cancer) in the presence of a large excess of wild type alleles. Detection of low-prevalence somatic mutations in tumors with heterogeneity, stromal contamination or in bodily fluids is difficult. However, the clinical significance of identifying these mutations is huge in many situations. For example, (a) in lung adenocarcinoma, low-level EGFR mutations that cannot be identified by regular sequencing can predict positive response to tyrosine kinase inhibitors or drug resistance; (b) mutations in plasma useful as biomarkers for early detection or tumor response to treatment cannot be sequenced using conventional methods; and (c) mutations in tumors with frequent stromal contamination, such as pancreatic or prostate cancer, can be 'masked' by presence of wild type alleles, thus requiring laborious micro-dissection or missing mutations altogether.

In some cases, minority alleles are microsatellites in which there are one or more deletions compared to microsatellites in majority alleles.

Microsatellites (e.g., homopolymer repeats) abide in the human genome. These comprise multiple repeats of a single or more nucleotides, such as poly-adenines (e.g., 15A's). A special type of mutation found in certain cancers involves insertions or deletions ('indels') in microsatellites, usually anywhere between 1-30 bp long. During cell division, polymerases performing DNA duplication often produce indels when they synthesize over microsatellites. These indels are often repaired by DNA mismatch repair enzymes. However, in many diseases (e.g., cancers), the indels found in microsatellites are increased due to mismatch repair deficiency. This leads to microsatellite instability (MSI). High levels of MSI are predictive for outcome during chemotherapy for various types of cancers (e.g., colorectal cancer (CRC)) and have been associated with distinct characteristics and favorable results including better prognosis, a higher 5-year survival, and lesser metastasis. Tumors with MSI are also more amenable to treatment via immunotherapy. Hence it is of great interest to screen tumors for MSI. At the same time, monitoring of MSI in plasma by screening tumor circulating DNA instead of testing the tumor can be clinically valuable for prognostic or predictive applications or as a marker for assessment of residual tumor load, to detect minimal residual disease (MRD).

However, detection of MSI and microsatellite indels is difficult. For example, while MSI is more frequent in colon cancers than other cancers, MSI detection in colonoscopy-obtained polyps, as well as in cfDNA, is frequently confounded by sensitivity issues due to co-existing excessive amounts of wild-type DNA. Improvements in sensitivity of MSI detection include utilization of long nucleotide repeats that display increased instability as compared to shorter repeats (Bacher et al., PLoS One, 10, e0132727). Further, COLD-PCR technology (Li et al. , Nat Med, 14, 579-584; Milbury et al., Clin Chem, 58, 580-589; and How-Kit et al., Hum Mutat, 34, 1568-1580) has recently been adapted to enrich altered microsatellites and suppress wild-type (WT) alleles for sensitive detection of single microsatellite sequences in the HSP110 microsatellite (How-Kit et al., Hum Mutat. 2018 Mar;39(3):441-453). This approach utilizes a `blocker' DNA oligonucleotide that blocks polymerase amplification by hybridizing to wild-type HSP110 microsatellites. In contrast, the blocker oligonucleotide prevents amplification of deletion-containing microsatellites since the blocker only hybridizes to the wild-type microsatellite alleles. The DNA blocker approach for enrichment of deletions at microsatellites provides an improvement in the sensitivity of detection for given microsatellite targets.

Despite these improvements, the enrichment of mutations via PCR is ultimately limited by polymerase-introduced errors (`stutter bands') (Ellegren et al., Nat Rev Genet, 5, 435-445; and Milbury et al., Clin Chem, 55, 632-640) that introduce wild-type allele changes indistinguishable from genuine indels. Thus, small indels comprising few nucleotide changes unavoidably fall within stutter bands that confound interpretation when capillary electrophoresis is employed for endpoint detection. High resolution melting-based MSI detection enables convenient assessment of MSI, but is also PCR-based and liable to stutter artifacts. Similarly, sequencing (e.g., Next generation sequencing (NGS)) is error-prone when it comes to identifying changes in microsatellites. While bioinformatic approaches provide opportunities to correct or over-look polymerase and sequencing errors, detection of small indels within large homopolymers remains a problem that limits the otherwise highly promising NGS-based detection of MSI.

### INTRODUCTION

This disclosure provides compositions and methods for using a modified version of Nuclease-assisted Mutation Enrichment (NaME) to enrich microsatellites containing deletions relative to wild-type microsatellites that do not contain deletions. The methods disclosed herein enable simultaneous enrichment of deletion-containing microsatellites at multiple targets or throughout the entire genome (g- NaME) as opposed to previous approaches that enrich indels in one or few DNA sequences or microsatellites at a time. This high throughput approach is anticipated to enable an unprecedented increase in sensitivity and the ability to detect presence of microsatellite deletions with a limited amount of whole-genome sequencing. This approach has unique applications such as detection of minimal residual disease by screening circulating DNA in blood samples obtained from patients that underwent therapy. Alternatively, methods disclosed herein can be used for early cancer detection in patients that do not have signs of disease.

Methods provided herein allow for multiplexed enrichment of microsatellites having deletions in a way that does not require knowledge of sequence of either microsatellites or sequences flanking microsatellites.

Contemplated herein is the use of NaME or NaME-with probe overlap (NaME-PrO) (Nucleic Acids Res. 2016 Nov 2; 44(19): e146) with generic probes. Generic probes, as described herein, comprise of a region that is complementary to a microsatellite sequence and which may be flanked by nucleotides that base-pair without specificity (e.g., inosines). The nucleotides of the probes that flank the region that is complementary to a microsatellite sequence do not form a sequence that is specifically complementary to sequences flanking microsatellites in genomic DNA. Herein, "flanking" is used to mean that there is no nucleotide between the sequence being flanking and the sequence/nucleotides that is flanking.

### SUMMARY OF THE INVENTION

The invention is set out in the appended claims. Specifically, provided is a method for enriching deletion-containing microsatellite targets in a sample of genomic DNA, the method comprising:
providing a nucleic acid sample comprising:
   at least a first double-stranded wild-type nucleic acid containing a microsatellite and at least a first double-stranded target nucleic acid suspected of containing a microsatellite corresponding to the wild-type microsatellite with at least one deletion relative to the wild-type microsatellite;
   adding to the nucleic acid sample a pair of oligonucleotide probes comprising of a first probe and a second probe, wherein the first probe comprises a region that is complementary to the microsatellite sequence in the wild-type nucleic acid top strand and the second probe comprises a region that is complementary to the microsatellite sequence in the wild-type nucleic acid bottom strand; wherein the length of the region that is complementary to the microsatellite sequences is at least 5 nucleotides;
   adding to the nucleic acid sample a double strand specific nuclease (DSN);
   subjecting the nucleic acid sample to a first temperature that destabilizes or denatures the at least first double-stranded wildtype and the at least first target mutant nucleic acids;
subjecting the nucleic acid sample to a second temperature that allows preferential formation of complementary wild-type nucleic acid-probe duplexes relative to partially complementary target nucleic acid-probe duplexes; and
subjecting the nucleic acid sample to a third temperature that allows the DSN to preferentially cleave the complementary wild-type nucleic acid-probe duplexes relative to partially complementary target nucleic acid-probe duplexes, wherein the DSN is added:
(i) after subjecting the nucleic acid sample to the second temperature, or
(ii) before subjecting the nucleic acid sample to the first temperature, and wherein the method further comprises adding to the nucleic acid sample an organic solvent that lowers the melting temperature of the wild-type and target nucleic acids to inhibit inactivation of the DSN when the nucleic acid sample is subjected to the first temperature.

In some embodiments, the third temperature is higher than the melting temperature of the target nucleic acid-probe duplexes.

In some embodiments, a DSN is thermostable.

In some embodiments, a first and a second oligonucleotide probes each comprise at least one modified nucleotides (e.g., locked nucleotide (LNA), peptide nucleic acid (PNA), or xeno nucleic acid (XNA)) that increases the difference in melting temperatures of the fully-matched wild-type nucleic acid-probe sequence and target nucleic acid-probe mismatched sequences

In some embodiments, a first and a second oligonucleotide probes each comprise 2 to 5 modified nucleotides (e.g., LNAs, PNAs, or XNAs), wherein the distance between the innermost LNAs, PNAs, or XNAs is greater than 10 nucleotides. In some embodiments, the region of the first and second probes that is complementary to the microsatellite sequence in the top and bottom strands of the wild-type nucleic acid is 5-100 nucleotides long. In some embodiments, a first and second probes comprise 1-20 inosines that flank one or both sides of the region that is complementary to the microsatellite sequence. In some embodiments, the number of inosines in the 5' end of the first probe is not equal to the number of inosines in the 3' end of the second probe, and the number of inosines in the 3' end of the first probe is not equal to the number of inosines in the 5' end of the second probe. In some embodiments, a first and second probes are 5 to 150 nucleotides long. In some embodiments, a first and second probes are 5 to 190 nucleotides long.

In some embodiments, a method for enriching deletion-containing microsatellite targets using probes further comprises adding Mg²⁺ to the nucleic acid sample so that the concentration of the Mg²⁺ in the nucleic acid sample is between 15-25mM when the nucleic acid sample is subjected to the second temperature and/or third temperature.

In some embodiments, a microsatellite is a mono-nucleotide repeat, di-nucleotide repeat, or tri-nucleotide repeat.

In some embodiments, a first and second probes are in molar excess of 100-fold to 1 billion-fold compared to wild-type and target nucleic acids.

### BRIEF DESCRIPTION OF THE DRAWINGS

The following drawings form part of the present specification and are included to further demonstrate certain aspects of the present disclosure, which can be better understood by reference to one or more of these drawings in combination with the detailed description of specific embodiments presented herein. It is to be understood that the data illustrated in the drawings in no way limit the scope of the disclosure.
FIG. 1 shows preferential, genome-wide enrichment of deletion-containing microsatellites (e.g., homopolymers) using generic oligonucleotide probes.
FIG. 2A shows a generic oligonucleotide probe design, which can optionally have modified nucleotides (e.g., LNAs, PNAs, and/or XNAs) at the two ends. SEQ ID NO: 1 provides an example of a probe with modified nucleotides (e.g., LNAs, PNAs, and/or XNAs).
FIG. 2B shows the working of the probe shown in FIG. 2A. Two ends of the oligonucleotide probe may contain modified nucleotides, such that stronger bonds are generated at the two ends. If there is a deletion anywhere, one of two ends will create no bond, thereby reducing the chance of digestion by DSN. SEQ ID NO: 1 and SEQ ID NO: 2 provides an example of a probe and a sequence to which the probe is complementary, respectively.
FIG. 3A shows a generic oligonucleotide probe (with a sequence of SEQ ID NO: 1) design with inosines (X) or other nucleotides that enable generic base-pairing to sequences on DNA that flank microsatellites.

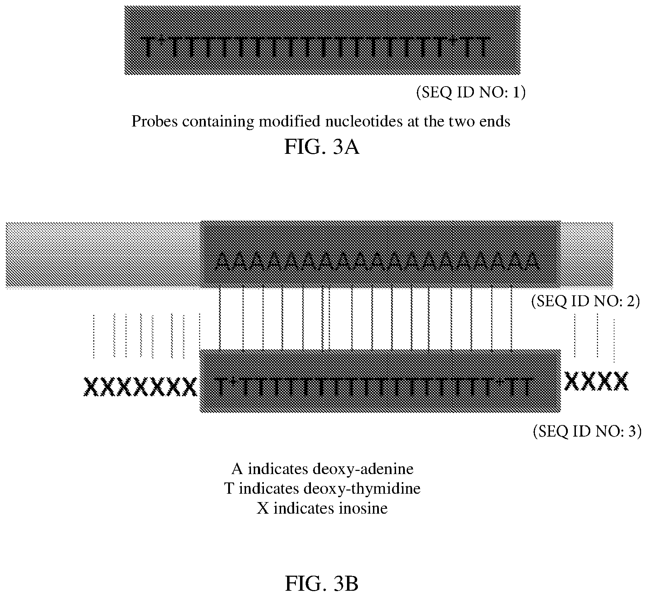

FIG. 3B shows the working of the probe shown in FIG. 3A. The two ends of the oligonucleotide probe may contain additional inosines on either end. Inosines are able to base-pair with any DNA base. Consequently they will bind to the nucleotides flanking the microsatellites on either side, thereby increasing the melting temperature Tm of the probe and enabling hybridization at optimal temperatures where DSN digestion is most active 60-65°C. SEQ ID NO: 3 and SEQ ID NO: 2 provide examples of sequences of a probe having inosines on both ends and a sequence to which the probe is complementary, respectively.
FIG. 4 shows preferential, genome-wide enrichment of deletion-containing microsatellites (e.g., homopolymers) without the use of oligonucleotide probes ( 'self-NaME').
FIG. 5 shows an overall process comprising enzymatic-based elimination of wild-type microsatellites at multiple genomic targets.
FIG. 6 shows high-resolution melting data for NaME PrO without probes (self-NaME) with 1 NaME-PrO round and 2 rounds at 54°C using dilutions of cell-line mixtures. The experiments compare wild-type (HMC DNA) and DNA from cell lines that contain deletions in microsatellites.
FIG. 7 shows high-resolution melting data for NaME PrO without probes with 1 NaME-PrO round and 2 rounds at 54°C using dilutions of cell-line mixtures (fragmented DNA, 30ng). The experiments compare wild-type (HMC DNA) and DNA from cell lines that contain deletions in microsatellites.
FIG. 8 shows high-resolution melting data for NaME (BAT25) without probes using 48ng of 8.3%, 3% and 1% of each cell line mixture at 54°C using 0.5U DSN. The results show High Resolution Melting comparing wild-type and DNA from cell lines that contain deletions in microsatellites. The deletion (shown by arrow) can be seen at lower dilutions of the deleted DNA when using NaME, as compared to no NaME.
FIG. 9 shows high-resolution melting data for NaME using generic probes containing inosines at the two ends to enrich a randomly chosen microsatellite (#140). The deletion (shown by arrow) can be seen at lower dilutions of the deleted DNA when using generic probes plus NaME, as compared to no NaME. Further, generic probes work as well as sequence specific probes.
FIG. 10A shows the effect of increasing the Mg++ concentration on NaME-PrO enrichment using 15A inosine probe plus 15T partially-complementary probe for the opposite strand. Assessment of enrichment on a randomly chosen target, poly-A15 #140 is shown. A 5% or 1% mix of MSI-positive cell line mix into WT DNA was tested via capillary electrophoresis after NaME-PrO application. By increasing the Mg⁺⁺ concentration to 15mM and 25mM, an increase in the enrichment of the small deletions that can be detected via capillary electrophoresis (arrows) is observed.
FIG. 10B shows the design of the universal 15A probe with flanking insosines (that bind generically to all nucleotides; having a sequence of SEQ ID NO: 4) and 15T probe with flanking inosines (having a sequence of SEQ ID NO: 5) that was used in experiments, data of which is shown in FIG. 15A. The standard Mg⁺⁺ is 5mM. Deletions can be observed down to 1% mutant to WT ratio.
FIG. 11 shows non enzymatic-based elimination of wild-type microsatellites at multiple genomic targets in a process comprising COLD-PCR.
FIG. 12 shows non enzymatic-based selection of A:T containing sequences via genome-wide amplification using COLD-PCR.

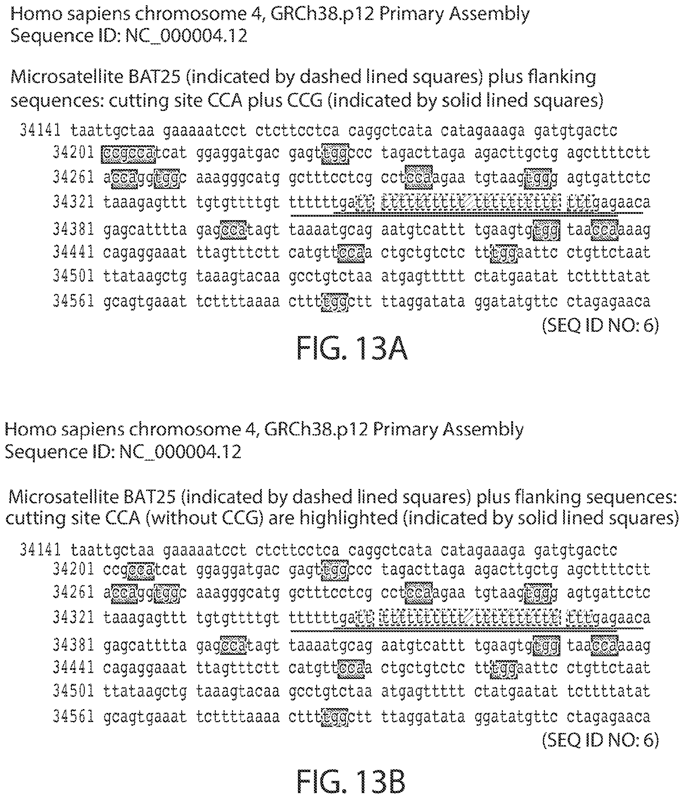

FIGs. 13A-13B show anticipated restriction sites for enzyme CviPII in the sequences containing the microsatellite BAT25 (dashed lined squares). There are no cutting sites on the microsatellite, while there are numerous cutting sites in the flanking region (and everywhere else in the genome except for microsatellites that lack the recognition sequences, CCA or CCG for top and bottom strands). FIG. 13A illustrates when the DNA is unmethylated, which allows CCG to be cut. FIG. 13 B illustrates when the DNA is methylated (e.g., via DNA methyltransferase treatment, or naturally), which prevents CCG (SEQ ID NO: 6) from being cut.

### DETAILED DESCRIPTION OF THE INVENTION

Nuclease-assisted Mutation Enrichment (NaME) that results in selective degradation of target wild type DNA or RNA, thereby providing enrichment of mutated target sequences has been previously disclosed in WO 2016/210224 and child applications thereof. NaME utilizes a double-strand specific nuclease (DSN, e.g., from crab or shrimp) that selectively degrades double stranded nucleic acid (e.g., DNA, or DNA/RNA hybrids), while it has minimal action on single stranded nucleic acid (e.g., ssDNA or ssRNA).

NaME can be used before, during or after PCR or other amplification methods. Subsequently, mutation-enriched sequences can be screened via any currently available method to identify the mutations (e.g., Sanger Sequencing, SSCP, next generation sequencing, MALDI-TOF for known mutations, High Resolution Melting HRM for pre-screening unknown mutations, and Single Molecule Sequencing, or third generation sequencing).

An adaptation of NaME to detect microsatellites on specific DNA targets is described in Ladas et al., Nucleic Acids Res, 46, e74. By using NaME before PCR, wild-type microsatellites are eliminated before generation of the `stutter bands', hence bypassing the false positives generated by such polymerase errors. Thus, NaME was shown to be an excellent way to eliminate wild type microsatellites, thereby enriching microsatellites with deletions and thus enhancing the detection of indel-containing microsatellites without being affected by stutter bands. This NaME approach for enriching specific micro-satellite containing targets having deletions uses probes that have a region that is complementary to a microsatellite and which also has sequences flanking the region that is complementary to the microsatellite, wherein the flanking sequence is complementary to the sequence in a wild-type nucleic acid that flanks the microsatellite.

Herein, novel methods of enriching microsatellites with deletions (herein referred to as "target microsatellites") are provided in which either generic probes (that do not comprise sequences that flank a region that is complementary to microsatellites that are complementary to sequence on wild-type nucleic acids that flank the microsatellites) or no probes are used in NaME so that multiple target microsatellites are enriched in a sample of genomic nucleic acid simultaneously.

The applications and field of use of the presently-disclosed method include any enrichment of any target sequence in a manner that achieves very high multiplexity in a single tube reaction, i.e. it is effectively a genome-wide enrichment for microsatellite repeat sequences with deletions. Thus g-NaME can be used for a very high sensitivity detection of deletions in microsatellites over the whole genome, thereby increasing sensitivity and reducing the amount of sequencing and sequencing reagents that need to be consumed to identify deletions present in samples that have a low level of target sequence to be detected, e.g., liquid biopsies in cancer patients. Highly sensitive detection of minimal residual disease in cancer patients can be achieved by use of the method disclosed herein. By applying g-NaME to tumors, it is possible to identify tumor-specific deletions present at microsatellites (e.g., homopolymers) and then trace these to the corresponding circulating DNA, thereby identifying minimal residual disease with high sensitivity and low-pass sequencing, using minimal amount of circulating DNA.

If genome-wide sequencing is performed without any enrichment, some deletions might still be detected albeit at lower sensitivity and with the requirement of deeper and more expensive sequencing. Therefore, provided herein is a method of improving the sensitivity of detecting deletion-containing microsatellite targets in a sample of genomic DNA.

### gNaME using generic oligonucleotide probes

The invention as claimed is directed to a method for enriching deletion-containing microsatellite targets in a sample of genomic DNA, the method comprising:
providing a nucleic acid sample comprising:
   at least a first double-stranded wild-type nucleic acid containing a microsatellite and at least a first double-stranded target nucleic acid suspected of containing a microsatellite corresponding to the wild-type microsatellite with at least one deletion relative to the wild-type microsatellite;
   adding to the nucleic acid sample a pair of oligonucleotide probes comprising of a first probe and a second probe, wherein the first probe comprises a region that is complementary to the microsatellite sequence in the wild-type nucleic acid top strand and the second probe comprises a region that is complementary to the microsatellite sequence in the wild-type nucleic acid bottom strand; wherein the length of the region that is complementary to the microsatellite sequences is at least 5 nucleotides;
   adding to the nucleic acid sample a double strand specific nuclease (DSN);
   subjecting the nucleic acid sample to a first temperature that destabilizes or denatures the at least first double-stranded wildtype and the at least first target mutant nucleic acids;
subjecting the nucleic acid sample to a second temperature that allows preferential formation of complementary wild-type nucleic acid-probe duplexes relative to partially complementary target nucleic acid-probe duplexes; and
subjecting the nucleic acid sample to a third temperature that allows the DSN to preferentially cleave the complementary wild-type nucleic acid-probe duplexes relative to partially complementary target nucleic acid-probe duplexes, wherein the DSN is added:
(ii) after subjecting the nucleic acid sample to the second temperature, or
(ii) before subjecting the nucleic acid sample to the first temperature, and wherein the method further comprises adding to the nucleic acid sample an organic solvent that lowers the melting temperature of the wild-type and target nucleic acids to inhibit inactivation of the DSN when the nucleic acid sample is subjected to the first temperature.

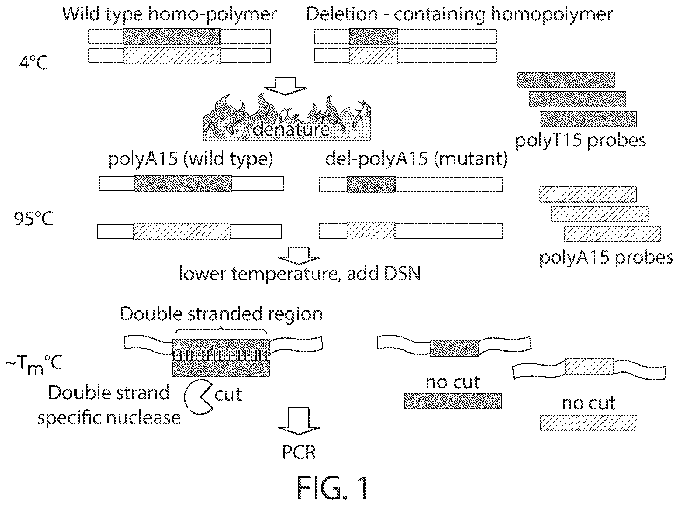

The oligonucleotides probes are designed to have a region that is complementary to at least part of a microsatellite in wild-type nucleic acid and this region. These generic-design probes, targeting both the sense and the antisense DNA strands can be added to the reaction prior to denaturation and addition of the DSN enzyme (see e.g., FIG. 1). Generic-design NaME probes are designed for both the sense and the anti-sense DNA strands, (e.g., for a homopolymer comprising adenosines and thymine, polyA probes plus polyT probes).

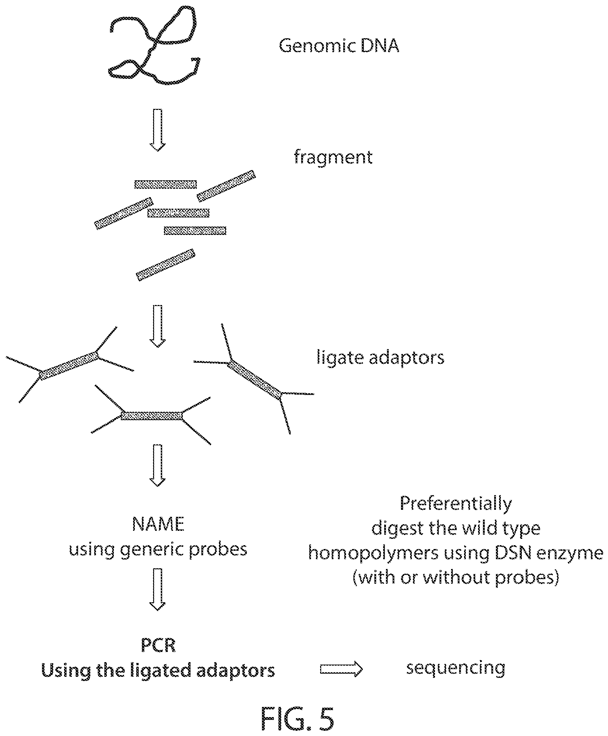

The following provides an example of the method. By using probes in high excess relative to the target nucleic acid (e.g., 100-fold or 1000-fold or more molar excess to the poly-A sequences present in the sample), the deletion enrichment using this embodiment is anticipated to be more efficient than without using probes. E.g. for polyA15 microsatellites comprising 15 sequential adenines the perfect match is poly-15T; and so on. Following the denaturation step, the temperature is lowered to a temperature at or below the Tm of the probe added and DSN enzyme is added to the solution. If there is a deletion in a polyA15, the probe will not bind well, since the Tm of the target-probe duplex will be lower than the Tm of the full complement, 15A binding to 15T. Accordingly, the wild type poly-A15 microsatellites will form duplexes with poly-T15 and be preferentially digested by DSN relative to microsatellites having a deletion that duplex with the poly-T15 on partially. Following PCR amplification of the intact sequences the deletion-containing polyA will be preferentially enriched over the wild type alleles. FIG. 5 provides an example of an overall process from the starting genomic DNA to sequencing.

The nucleic acid sample is of genomic DNA. In some embodiments, genomic DNA is isolated from a biological sample (e.g., blood, plasma, serum, urine, CSF, buccal-swab, or solid tissue (e.g., a tumor tissue)). In some embodiments, genomic DNA is cell-free circulating DNA. In some embodiments, genomic DNA as provided in a sample of nucleic acid is fragmented. In some embodiments, DNA fragments are 20-5000 bp long (e.g., 20-100, 20-150, 50-150, 50-200, 100-500, 100-1000, 100-2000, 500-2000, or 1000-5000 bp long).

In some embodiments, a sample of DNA is suspected of containing at least one microsatellite. In some embodiments, a sample of DNA is suspected of containing at least one microsatellite with one or more insertions or deletions. A microsatellite is a tract of repetitive DNA in which certain DNA motifs (ranging in length from one to six or more base pairs) are repeated, typically 5-50 times. Microsatellites occur at thousands of locations within an organism's genome. They have a higher mutation rate than other areas of DNA. Microsatellites are also called short tandem repeats (STRs) or simple sequence repeats (SSRs). Microsatellites as referred to as herein also include mini satellites that are of larger length (e.g., up to 100 bp). Microsatellites in a sample of nucleic acid can be of multiple types and of varying length. Non-limiting examples of microsatellites are mono-nucleotide repeats (e.g., AAAAAAAAA), di-nucleotide repeats (e.g., ACACACACACACACA (SEQ ID NO: 7)), or tri-nucleotide repeats (e.g.,CAGCAGCAGCAGCAGCAG (SEQ ID NO: 8)). In some embodiments, a microsatellite has a repeat of more than three nucleotides. A single sample of DNA can have mono-nucleotide repeats, di-nucleotide repeats, and/or trinucleotide repeats, each of varying length. For example, a sample of nucleic acid may comprise a poly-A repeat that is of 15 bp, 18bp, 25bp, and 40 bp. It may also comprise CAG repeats of multiple lengths.

In some embodiments, a microsatellite on wild-type nucleic acids is 5-100 (e.g., 5-100, 5-10, 10-20,10-30, 10-50, 20-30, 20-25, 20-40, 30-50, 20-60, 40-60, 50-100, 50-80, 50-70, or 80-100) bp or nucleotides long. In some embodiments, a microsatellite is at least 5 (e.g., at least 5, at least 10, at least 12, at least 15, at least 20, at least 25, at least 30, at least 40, at least 50, at least 60, at least 70, at least 80, at least 82, at least 84, at least 86, at least 88, at least 90, at least 92, at least 94, at least 96, or at least 98) bp or nucleotides long.
In some embodiments, a microsatellite on wild-type nucleic acids is 5-100 (e.g., 5-100, 5-10, 10-20,10-30, 10-50, 20-30, 20-25, 20-40, 30-50, 20-60, 40-60, 50-100, 50-80, 50-70, or 80-100) repeats long. For mono-nucleotide repeats, a repeat is one nucleotide long. For di-nucleotide repeats, a repeat is two nucleotides long. For tri-nucleotide repeats, a repeat is three nucleotides long. Therefore, a microsatellite with di-nucleotide repeats having the same number of repeats as a microsatellite with mono-nuclear repeats will be twice as long as the microsatellite with mono-nuclear repeats.

In some embodiments, a microsatellite is at least 5 (e.g., at least 5, at least 10, at least 12, at least 15, at least 20, at least 25, at least 30, at least 40, at least 50, at least 60, at least 70, at least 80, at least 82, at least 84, at least 86, at least 88, at least 90, at least 92, at least 94, at least 96, or at least 98) repeats long.

A deletion in a microsatellite may be up to 95 bp long (e.g., up to 5, up to 10, up to 15, up to 20, up to 25, up to 30, up to 35, up to 40, up to 45, up to 50, up to 55, up to 60, up to 65, up to 70, up to 75, up to 80, up to 85, up to 90, or up to 95 bp long). In some embodiments, a deletion is one or more than 1 bp (e.g., 1, more than 1, more than 2, more than 5, more than 10, more than 15, more than 20, more than 25, more than 30, more than 35, more than 40, more than 45, more than 50, more than 55, more than 60, more than 65, more than 70, more than 75, more than 80, more than 85, more than 90, more than 95, more than 100, more than 105, more than 110, more than 115, more than 120, more than 125, or more than 135 bp).

Oligonucleotides probes as used in the method are also referred to as generic oligonucleotide probes. Generic oligonucleotide probes do not comprise any sequence that is complimentary to a sequence flanking the microsatellite. They are complementary to and bind all microsatellites that are as long as or longer than the probe. They are generic because they bind to multiple microsatellites, each at a different location in the genome. They are not specific to a single sequence in the genome. As described below, generic probes can include inosines flanking the portion complementary to the microsatellite. The inosine may then bind portions of the microsatellite not bound by the microsatellite specific sequences or bind to regions flanking the microsatellite but not part of the repeating sequence of the satellite. Oligonucleotide probes as used here are described in more detail below. In some embodiments, a pair of oligonucleotide probes comprise of a first probe and a second probe, wherein the first probe comprises a region that is complementary to a microsatellite sequence in a first wild-type nucleic acid top strand and the second probe comprises a region that is complementary to a first microsatellite sequence in the wild-type nucleic acid bottom strand. In some embodiments, the first and second probes are complementary to each other. In some embodiments, the length of the region that is complementary to the microsatellite sequences is at least n nucleotides, wherein n is 5-100 (e.g., 5-100, 5-10, 10-20,10-30, 10-50, 20-30, 20-25, 20-40, 30-50, 20-60, 40-60, 50-100, 50-80, 50-70, or 80-100) nucleotides long. In some embodiments, oligonucleotides probes are added so that they are in molar excess compared to wild-type and target nucleic acids. In some embodiments, probes are in molar excess of 100-fold to 1 billion-fold (e.g., 100-fold, 1000-fold, 10,000-fold, 100,000-fold, 1,000,000-fold, 10,000,000-fold, 100,000,000, or 1billion-fold) compared to wild-type and target nucleic acids.

The method of enriching deletion-containing microsatellite targets comprises adding to the nucleic acid sample a double strand specific nuclease (DSN), also referred to as duplex-specific nuclease. In some embodiments, a DSN is sourced from crabs. In some embodiments, a DSN is sourced from shrimp. DSNs are enzymes that preferentially digest or cleave nucleotides that are duplexed, e.g., dsDNA, or dsDNA-RNA hybrid duplexes. In some embodiments, DSNs are thermostable so that they do not get deactivated at higher temperatures (e.g., above 80°C or 90°C) and/or under certain conditions. Activity of DSN can be measured using a Kunitz assay (Biosens Bioelectron. 2011; 26 (11):4294-300). One unit of DSN activity can be defined as the amount of DSN added to 50 µg/ml calf thymus DNA that causes an increase of 0.001 absorbance units per minute; Activity assay was performed at 25°C, in 50 mM Tris-HCl buffer, pH 7.15, containing 5 mM MgCl₂.

In some embodiments, the sample of nucleic acid is denatured to form ssDNA from dsDNA.

In some embodiments, the sample of nucleic acid is only destabilized. Destabilization of wild-type and target nucleic acids permits hybridization of the probes to their corresponding sequences on the wild type and mutant nucleic acids thereby forming complementary wild-type- probe duplexes on top and bottom strands, and partially complementary mutant-probe duplexes. By "destabilizing" it is meant that the double stranded wild type and target mutant nucleic acids denature to such an extent so as to allow the probes to hybridize to their corresponding sequences, but the wild type and target mutant nucleic acids do not denature completely. A condition that destabilizes is an increased temperature (e.g., 65°C - 80°C including 65°C, 70°C, 75°C, 80°C). This destabilizing temperature is typically about 10-20°C below the melting temperature (Tm) of the nucleic acid sequence. At this temperature, the oligonucleotide probes invade and bind to their corresponding sequences on the wild type and mutant nucleic acids. The probes fully match the sequences on the wild type nucleic acid and can, thus, form complimentary wild type probe duplexes (i.e., with no mis-matches). Therefore, the first temperature is one that either destabilizes the wild-type and target nucleic acids, or denatures them. The second temperature is a temperature at which probes hybridize to wild-type and target nucleic acids to form complementary wild-type nucleic acid-probe duplexes or partially complementary target nucleic acid-probe duplexes. In some embodiments, the second temperature is the melting temperature or slightly below (up to 5°C) the melting temperature of wild-type nucleic acid-probe duplexes. In some embodiments, the second temperature is above the melting temperature or slightly above (up to 5°C) the melting temperature of target nucleic acid-probe duplexes.

The third temperature is a temperature at which DSN is active and digests duplexed sequence.

The solvent lowers the Tm of the nucleic acids, without inhibiting or diminishing the activity of DSN. In some embodiments, addition of solvent lowers the Tm of the nucleic acids allowing the first temperature for denaturation/destabilization of the nucleic acids to be lower, such that there is lower degree of deactivation of the DSN. Examples of such solvents include, but are not limited to DMSO, betaine or formamide. In some embodiments, 10-15% of DMSO is included in the reaction mixture or nucleic acid sample. In some embodiments, DSN is added after subjecting the nucleic acid sample to the second temperature. In some embodiments, a thermostable DSN is used that can withstand a higher temperature without its activity being diminished either partially or completely.

In some embodiments, once a nucleic acid sample is subjected to a third temperature to allow the DSN to digest dsDNA, the DSN is then inactivated either partially or completely, for example, by heating the sample to 95°C for 1-10 min.

In some embodiments, DSN is added so that its concentration in the reaction is 0.01-10 units (e.g., 0.01, 0.1, 0.2, 0.3, 0.4, 0.5, 0.6, 0.7, 0.8, 0.9, 1, 1.5, 2, 3, 4, 5, 6, 7, 8, 9, or 10 units).

In some embodiments, the Mg²⁺ concentration in the reaction mixture is adjusted so that it is between 10-30 mM (e.g., 15-25mM). In some embodiments, Mg²⁺ concentration is adjusted before subjecting the sample to the first temperature. In some embodiments, Mg²⁺ concentration is adjusted before subjecting the sample to the second temperature.

In some embodiments, a single reaction is performed using more than one pair of probes (e.g., 2, 3, 4, 5, 6, 7, 8, 9,10 or more pairs of probes), wherein each pair of probe has a sequence that is different from that of the other pairs of probes. For example, a first pair of probes may have a region that is complementary to a poly-A repeat, while a second pair of probes may have a region that is complementary to a poly-G repeat, while a third pair of probes may have a region that is complementary to a poly-AT repeat.

In some embodiments, a sample if nucleic acid suspected of comprising a target nucleic acid is subjected to multiple NaME reactions (e.g., 1, 2, 3, 4, 5, 6, 7, 8, 9, 10, or more than 10 reactions), each reaction conducted with a different pair of probes. In some embodiments, the pairs of probes in a first reaction differ from a pair of probes in a second reaction only by the length of the region that is complementary to wild-type nucleic acids. For example, a pair of probes in a first reaction and the second reaction may comprise first and second probes of poly-A and poly-T sequence, example that the probes in the first reaction have a region that is complementary to the wild-type strands that is 15 nucleotides long, whereas the probes in the second reaction have a region that is complementary to the wild-type strands that is 20 nucleotides long. In some embodiments, the sequence of probes in a first and second reaction are different. For example, the probes of a first reaction may comprise a region that is complementary to a mono-nucleotide repeat and the probes of a second reaction may comprise a region that is complementary to a di-nucleotide repeat or a mono-nucleotide repeat that is different from the mono-nucleotide repeat of the probes of the first reaction.

### Oligonucleotide probes

Provided herein are generic oligonucleotide probes. In some embodiments, generic probes are provided in a pair comprising a first and second probe, wherein the first probe and second probe each have a region that is complementary to the top and bottom strands of wild-type microsatellite sequences. The regions of the first and second probes that are complementary to microsatellite sequences may be flanked by nucleotides, however these nucleotides do not form a sequence that is specifically complementary to sequences that flank microsatellite sequences on wild-type nucleic acids. Herein, "flanking" is used to mean that there is no nucleotide between the sequence being flanking and the sequence/nucleotides that is flanking.

As describes above, generic oligonucleotide probes do not comprise any sequence that is complimentary to a sequence flanking the microsatellite. They are complementary to and bind all microsatellites that are as long as or longer than the probe. They are generic because they bind to multiple microsatellites, each at a different locations in the genome. They are not specific to a single sequence in the genome. As described below, generic probes can include inosines flanking the portion complementary to the microsatellite. The inosine may then bind portions of the microsatellite not bound by the microsatellite specific sequences or bind to regions flanking the microsatellite but not part of the repeating sequence of the satellite.

In some embodiments, generic probes, as described herein, comprise of a region that is complementary to a microsatellite sequence and which may be flanked by nucleotides that base-pair without specificity (e.g., inosines). The nucleotides of the probes that flank the region that is complementary to a microsatellite sequence do not form a sequence that is specifically complementary to sequences flanking microsatellites in genomic DNA. Herein, "flanking" is used to mean that there is no nucleotide between the sequence being flanking and the sequence/nucleotides that is flanking.

In some embodiments, generic probes, as described herein, consist of a region that is complementary to a microsatellite sequence and which may be flanked by nucleotides that base-pair without specificity (e.g., inosines). The nucleotides of the probes that flank the region that is complementary to a microsatellite sequence do not form a sequence that is specifically complementary to sequences flanking microsatellites in genomic DNA. Herein, "flanking" is used to mean that there is no nucleotide between the sequence being flanking and the sequence/nucleotides that is flanking.

Probes contain a region (S) that is complementary to the satellite, optionally flanked by inosines (I)1-20 and/or an adaptor (A). The probe is of the formula I*S*I, A*I*S*I*A, or A*S*A, where I is 1-20 inosines and * is a bond.

In some embodiments, a probe consists of a mono-nucleotide repeat, di-nucleotide repeat, or tri-nucleotide repeat, in each case optionally flanked at each end by 1-20 inosines.

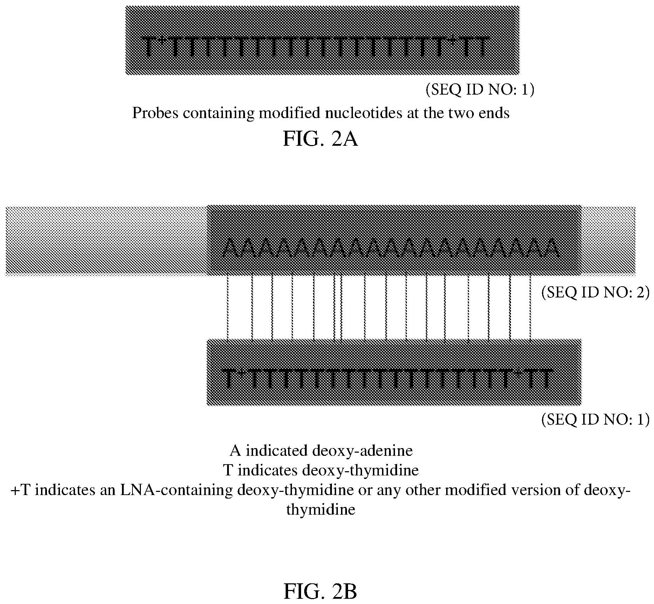

In some embodiments, the region that is complementary to the top strand of a first probe is complementary to the region that is complementary to the bottom strand of a second probe. FIG. 2A provides an example of a first probe that has a region that is complementary to the microsatellite sequence is the top strand of a wild-type nucleic acid (as shown in FIG. 2B).

In some embodiments, the region of a probe that is complementary to wild-type sequences is 5-100 (e.g., 5-100, 5-10, 10-20,10-30, 10-50, 20-30, 20-25, 20-40, 30-50, 20-60, 40-60, 50-100, 50-80, 50-70, or 80-100) nucleotides long. In some embodiments, the region of a probe that is complementary to wild-type sequences is 15, 20, 21,22, 23, 24, 25, 26, 27, 28, 29, or 30 nucleotides long.

Oligonucleotide probes for use in any one of the methods disclosed herein may comprise natural deoxynucleotides (DNA), or natural nucleotides (RNA), or modified deoxynucleotides or nucleotides. The modification may comprise one or more artificial nucleotides such as: locked nucleic acid (LNA), peptide nucleic acid (PNA), and xeno nucleic acid (XNA); or any other modified nucleotide that increases the difference in melting temperatures of the fully-matched wild-type nucleic acid-probe sequence and target nucleic acid-probe mismatched sequences. In a preferred embodiment, the modified nucleotides are placed at the two ends of the probe (see e.g., FIGs. 2A and 2B). As an example, in the same setting where poly-T probes are directed against poly-A microsatellites, if the LNA-containing poly-T15 binds to the corresponding wild type poly-A15 microsatellite the binding will be stronger than the equivalent natural nucleotide bonding and will have a higher Tm in view of the LNA-modified ends. In some embodiments, an LNA-containing T is placed one nucleotide from each end of the probe (or, in some embodiments, each end of the probe that is complementary to a microsatellite). In contrast, if there is a small deletion in a microsatellite, then the probes will not bind well, since only one of the two LNA-containing nucleotides will be binding, but not both. Hence the Tm of the hybrid with a deletion-containing microsatellite will be much lower and the duplex will not form. Accordingly, addition of the DSN enzyme will not digest such deletion containing sequences, which will then be enriched following PCR.

In some embodiments, each of the first and second probes of a pair of probes comprises at least one (e.g., at least one, at least two, at least three, at least four, at least five, at least six, or at least 7) modified nucleotide (e.g., LNA, PNA, or XNA) that increases the difference in melting temperatures of the fully-matched wild-type nucleic acid-probe sequence and target nucleic acid-probe mismatched sequences. In some embodiments, there is at least one (e.g., 1, 2, 3, 4, 5, 6, 7, 8, 9, or 10) modified nucleotide on each of the 5' and 3' ends of the regions that are complementary to wild-type strand. For example, there may be 1 modified nucleotide on the 5' end of the region that is complementary to a wild-type strand and two modified nucleotides in the 3' end of the region that is complementary to a wild-type strand. In some embodiments, a modified nucleotides (e.g., LNA, PNA, or XNA) is located on the first nucleotide of the 5'-end portion of the probe that is complementary to the microsatellite, and/or on the first nucleotide of the 3'-end portion of the probe that is complementary to the microsatellite. In some embodiments, a modified nucleotides (e.g., LNA, PNA, or XNA) is located on the second nucleotide of the 5'-end portion of the probe that is complementary to the microsatellite, and/or on the first and or second nucleotide of the 3'-end portion of the probe that is complementary to the microsatellite.

In some embodiments, a probe comprises only LNAs as modified nucleotides. In some embodiments, a probe may have more than one type of modified nucleotide, e.g., one LNA and two XNAs.

In some embodiments, the modified nucleotides are placed at the two ends of the probe so that both bind to wild-type sequence but only one or none modified nucleotides bind to target sequence. In some embodiments, the inner-most modified nucleotides on a probe is at least 5, at least 10, at least 12, at least 15, at least 20, at least 25, at least 30, at least 40, at least 50, at least 60, at least 70, at least 80, at least 82, at least 84, at least 86, at least 88, at least 90, at least 92, at least 94, at least 96, or at least 98 nucleotides apart.

In some embodiments, inosines are added on either side of the oligonucleotide probe (see e.g., FIGs. 3A and 3B). These inosines can base-pair with any DNA base, thereby enabling stronger binding to all microsatellites and their flanking sequences. This is advantageous since the optimal digestion by DSN is about 60-65°C, thereby requiring binding of the probe to flanking sequences as well to achieve optimal performance of the present protocol.

In some embodiments, probes targeting microsatellites on both the sense and antisense DNA strands are always added. To avoid probes for the sense strand binding to probes for the antisense strand, a different number of inosines are designed into the probe that is complementary to the top strand and the probe that is complementary to the bottom strand. Thus, the probes for the top and bottom strand will be non-overlapping. At the temperature close to the probe Tm, the sense and antisense strand probes would not be expected to bind substantially to each other using this design. In some embodiments, each of a first and second probe comprises 1-20 (e.g., 1, 2, 3, 4, 5, 6, 7,, 8, 9, 10, 11, 12, 13, 14, 15, 16, 17, 18, 19, or 20) inosines that flank either or both sides of the region that is complementary to the microsatellite sequence in a wild-type nucleic acid. In some embodiments, the number of inosines in the 5' end of the first probe is not equal to the number of inosines in the 3' end of the second probe, and the number of inosines in the 3' end of the first probe is not equal to the number of inosines in the 5' end of the second probe. For example, if the 5' end of a first probe has 3 inosines, then the 3' end of the second probe should not have 3 inosines; it may have 1, 2, 5, 6, 7, 8, or 9 inosines. This unequal pairing of number of inosines in the 5' and 3' ends of the first and second probes, or 3' and 5' ends of the first and second probes, respectively, ensures that the probes do not hybridize to each other.

In some embodiments, a probe is 5-190 (e.g., 5-10, 5-20, 10-20, 10-30, 20-30, 20-40, 25-50, 20-50, 30-60, 40-60, 40-80, 50-80, 5-50, 10-80, 50-90, 50-100, 20-100, 50-150, 100-150, 100-190, 150-190, 120-190, 50-190, or 5-190) nucleotides long with a region that is complementary to a wild-type microsatellite that is up to 150 nucleotides long and having up to 20 inosines on either or both ends flanking the region that is complementary to a wild-type microsatellite. A microsatellite in a wild-type sequence may be a mono-nucleotide repeat, dinucleotide repeat, or tri-nucleotide repeat. In some embodiments, a microsatellite has a repeat of more than three nucleotides.

The following sections entitled "Self-g NaME", "Non-enzymatic elimination of wild-type microsatellites at multiple genomic targets using blocking probes", "Non-enzymatic enrichment of A:T microsatellites at multiple genomic targets using COLD-PCR", and "Universal microsatellite target-enrichment using restriction endonucleases" disclose further methods for enriching deletion-containing microsatellite targets in a sample of genomic DNA. These methods are not encompassed by the invention as set out in the appended claims.

### Self-g NaME

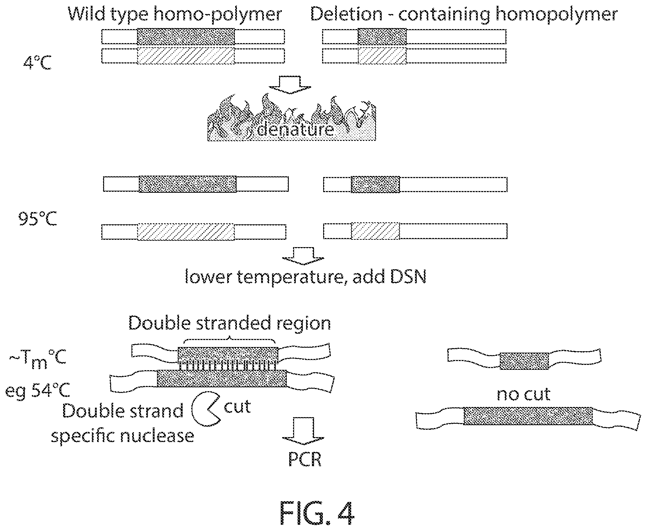

Self- NaME method for enriching and detecting microsatellite deletions takes advantage of DSN properties to preferentially degrade larger microsatellites and preserve short microsatellites such as those resulting from deletions occurring on wild-type (WT) microsatellites. It is similar to the method described herein which uses generic probes, however, does not use generic probes, and instead relies on hybridization of wild-type and target duplexes. See for example FIG. 4. Genomic DNA is first denatured (e.g., using a temperature of 95°C or higher), after which the temperature is reduced to an appropriate temperature just below the melting temperature (Tm) of the microsatellites being interrogated. For example, for 15A homopolymers, a hybridization temperature of 54°C is used. At this temperature the wild-type 15A homopolymers hybridize with other poly-adenine homopolymers in the genome and are digested by DSN. On the other hand, homopolymers containing deletions (e.g., a 10A homopolymer resulting from a 5A deletion on a wild-type 15A homopolymer) do not hybridize substantially at this temperature; being single-stranded, they are not digested by the DSN. Thereby, deletion-containing DNA is preferentially left un-degraded relative to corresponding wildtype microsatellites having no deletions. Hence a subsequent amplification (e.g., by PCR reaction) after DSN digestion is expected to amplify preferentially the deletion-containing alleles that remain substantially single stranded and unaffected by DSN. Because the self-hybridization takes place on all genomic homopolymer regions at once, thousands of deletions on homopolymers over the genome can be enriched at the same time, thus providing a highly multiplexed approach. Following enrichment, the deletion homopolymers can be detected via genome-wide sequencing.

Accordingly, also contemplated herein is a method for enriching deletion-containing microsatellite targets in a sample of genomic DNA, the method comprises:
providing a nucleic acid sample comprising:
   at least a first double-stranded wild-type nucleic acid containing a microsatellite and at least a first double-stranded target nucleic acid suspected of containing a microsatellite corresponding to the wild-type microsatellite with at least one deletion relative to the wild-type microsatellite;
adding to the nucleic acid sample a double strand specific nuclease (DSN);
subjecting the nucleic acid sample to a first temperature that denatures the at least first double-stranded wildtype and the at least first target mutant nucleic acids;
subjecting the nucleic acid sample to a second temperature that allows preferential formation of wild-type nucleic acid homo-duplexes relative to wild-type-target hetero-duplexes; and
subjecting the nucleic acid sample to a third temperature that allows the DSN to preferentially cleave the homo-duplexes relative to the hetero-duplexes.

When NaME is performed on samples having or suspected of having microsatellite targets without the use of generic probes, then the nucleic acid has to be denatured; destabilization will not work, because unless the nucleic acids are first denatured before allowed them to hybridize again, then the DSN will digest most of the nucleic acids.

### Non-enzymatic elimination of wild-type microsatellites at multiple genomic targets using blocking probes

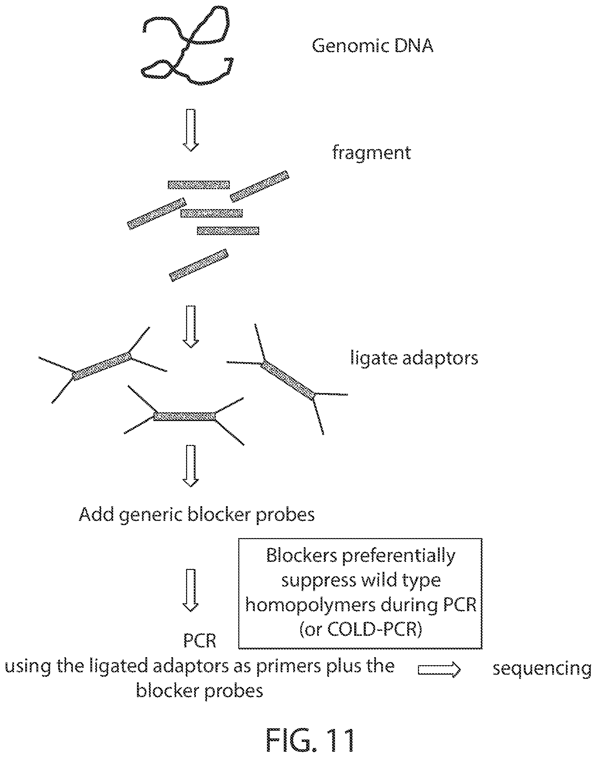

Also contemplated herein is a multi-site enrichment approach that does not require action of an DSN enzyme to suppress the wild-type microsatellites. FIG. 11 provides an illustration of this approach, in which oligonucleotide probes as described herein are used during amplification of nucleic acid strands using ligated adaptors, wherein the probes act as 'polymerase blockers' that preferentially bind to wild-type microsatellites and inhibit amplification. In contrast, when a microsatellite contains a deletion, the binding of the blocker probes to this microsatellite has a lower melting temperature (Tm), hence it does not bind well and allows polymerase extension and amplification. The amplification can be done using either standard PCR conditions, or COLD-PCR conditions. Any variation of COLD-PCR (e.g., temperature independent/tolerant COLD-PCR) can also be performed using the oligonucleotide probes disclosed herein. COLD-PCR and its derivatives, e.g., Temperature-Tolerant COLD-PCR are disclosed in the following patent application: US 2014/0051087, US 2016/0186237, US20180282798, US8455190, US20160186237.

Enrichment of microsatellite indels via COLD-PCR was described previously by How-Kit et al. (Hum Mutat. 2018 Mar;39(3):441-453). However, in How-Kit et al., the oligonucleotide blocker probes were designed to match a specific, unique sequence. In contrast the blocker probes employed in methods disclosed herein have a generic design that bind to multiple genomic sites ('genome-wide') as opposed to binding to a single unique sequence, thereby achieving genome-wide enrichment of indel-containing microsatellites.

Accordingly, further contemplated herein is a method for enriching deletion-containing microsatellite targets in a sample of genomic DNA, the method comprising:
(a) providing a nucleic acid sample comprising at least a first double-stranded wild-type nucleic acid containing a microsatellite and at least a first double-stranded target nucleic acid suspected of containing a microsatellite corresponding to the wild-type microsatellite with at least one deletion, and wherein each end of each strand of the double-stranded nucleic acids are ligated to an adaptor;
   providing a pair of oligonucleotide probes comprising of a first probe and a second probe, wherein the first probe comprises a region that is complementary to the microsatellite sequence in the wild-type nucleic acid top strand and the second probe comprises a region that is complementary to the microsatellite sequence in the wild-type nucleic acid bottom strand; wherein the length of the region that is complementary to the microsatellite sequences is at least 5 nucleotides;
   providing a pair of nucleic acid primers that are complementary to the adaptors and under amplification conditions can amplify the adapter ligated double-stranded nucleic acids;
(b) forming a reaction mixture containing the nucleic acid sample, the oligonucleotide probes, and the pair of nucleic acid primers;
(c) subjecting the reaction mixture to a temperature that denatures the at least first double-stranded wildtype and the at least first target mutant nucleic acids;
(d) subjecting the reaction mixture to a temperature that allows formation of complementary first wild-type nucleic acid-probe duplexes and partially complementary first target nucleic acid-probe duplexes, wherein the temperature is above the primer annealing/extension temperature;
(e) subjecting the reaction mixture to a first critical denaturation temperature (Tc) to permit preferential denaturation of first target nucleic acid-probe duplexes relative to first wild-type nucleic acid-probe duplexes, wherein the first Tc is below the lowest melting temperature of any first wild-type nucleic acid-probe duplexes;
(f) reducing the temperature of the reaction mixture in the presence of pairs of nucleic acid primers and permitting the primers to anneal to the adaptors, and
(g) extending the primers to enrich the target sequences.

Oligonucleotide probes can be any pair of generic oligonucleotides probes as disclosed herein. Adaptors are single-stranded or double-stranded and are at least 5 bp long (e.g., at least 5, at least 8, at least 10, at least 15, at least 20, at least 25, at least 30, or at least 35 bp long). In some embodiments, adaptors are ligated to one or more tag via which the adaptor can be immobilized. In some embodiments, a tag is biotin. In some embodiments, biotinylated adaptors, which are ligated to nucleic acid fragments are bound to streptavidin (e.g., on a bead).

The oligonucleotide probes as disclosed herein may also be used in temperature-independent COLD-PCR. Accordingly, a method of COLD-PCR using a first critical denaturation temperature is repeated using a second critical denaturation temperature that is higher than the first critical denaturation temperature. The method may further comprise repeating steps (e) to (g) at least once, wherein step (e) is repeated at a second Tc of a second double-stranded wild-type nucleic acid containing a microsatellite and a second double-stranded target nucleic acid suspected of containing a microsatellite corresponding to the wild-type microsatellite with at least one deletion target, the second Tc being above the first Tc and below the melting temperature of second wild-type nucleic acid-probe duplex to permit preferential denaturation of second target nucleic acid-probe duplexes relative to second wild-type nucleic acid-probe duplexes.

Instead of relying on critical denaturation temperature at which there is preferential denaturation of first target nucleic acid-probe duplexes relative to first wild-type nucleic acid-probe duplexes, a critical hybridization temperature may be relied upon to preferentially permit preferential hybridization of first wild-type nucleic acid-probe duplexes relative to first target nucleic acid-probe duplexes. Accordingly, also contemplated is a method comprising: (a) providing a nucleic acid sample comprising at least a first double-stranded wild-type nucleic acid containing a microsatellite and at least a first double-stranded target nucleic acid suspected of containing a microsatellite corresponding to the wild-type microsatellite with at least one deletion, and wherein each end of each strand of the double-stranded nucleic acids are ligated to an adaptor;
providing a pair of oligonucleotide probes comprising of a first probe and a second probe, wherein the first probe comprises a region that is complementary to the microsatellite sequence in the wild-type nucleic acid top strand and the second probe comprises a region that is complementary to the microsatellite sequence in the wild-type nucleic acid bottom strand; wherein the length of the region that is complementary to the microsatellite sequences is at least 5 nucleotides;
providing a pair of nucleic acid primers that are complementary to the adaptors and under amplification conditions can amplify the adapter ligated double-stranded nucleic acids;

(b) forming a reaction mixture containing the nucleic acid sample, the oligonucleotide probes, and the pair of nucleic acid primers;
(c) subjecting the reaction mixture to a temperature that denatures the at least first double-stranded wildtype and the at least first target mutant nucleic acids;
(d) subjecting the reaction mixture to a first critical hybridization temperature (Th) to permit preferential hybridization of first wild-type nucleic acid-probe duplexes relative to first target nucleic acid-probe duplexes, wherein the first Th is above the highest melting temperature of any first target nucleic acid-probe duplexes;
(e) reducing the temperature of the reaction mixture in the presence of pairs of nucleic acid primers and permitting the primers to anneal to the adaptors, and
(f) extending the primers to enrich the target sequences.

A method relying on a first critical hybridization temperature may further comprise repeating steps (d) to (f) at least once, wherein step (d) is repeated at a second Th of a second double-stranded wild-type nucleic acid containing a microsatellite and a second double-stranded target nucleic acid suspected of containing a microsatellite corresponding to the wild-type microsatellite with at least one deletion target, the second Th being below the first Th and above the melting temperature of second target nucleic acid-probe duplex to permit preferential hybridization of second wild-type nucleic acid-probe duplexes relative to second target nucleic acid-probe duplexes.

A method for enriching deletion-containing microsatellite targets in a sample of genomic DNA may involve simple PCR. Accordingly also contemplated here is a method comprising (a)providing a nucleic acid sample comprising at least a first double-stranded wild-type nucleic acid containing a microsatellite and at least a first double-stranded target nucleic acid suspected of containing a microsatellite corresponding to the wild-type microsatellite with at least one deletion relative to the wild-type microsatellite, and wherein each end of each strand of the double-stranded nucleic acids are ligated to an adaptor;
providing a pair of oligonucleotide probes comprising of a first probe and a second probe, wherein the first probe comprises a region that is complementary to the microsatellite sequence in the wild-type nucleic acid top strand and the second probe comprises a region that is complementary to the microsatellite sequence in the wild-type nucleic acid bottom strand; wherein the length of the region that is complementary to the microsatellite sequences is at least 5 nucleotides;
providing a pair of nucleic acid primers that are complementary to the adaptors and under amplification conditions can amplify the adapter ligated double-stranded nucleic acids;

(b) forming a reaction mixture containing the nucleic acid sample, the oligonucleotide probes, and the pair of nucleic acid primers;
(c) subjecting the reaction mixture to a temperature that denatures the at least first double-stranded wildtype and the at least first target mutant nucleic acids;
(d) reducing the temperature of the reaction mixture in the presence of pairs of
nucleic acid primers and pairs of oligonucleotide probes and permitting the primers and probes to anneal to the target sequences, wherein preferentially binding of probes to wild-type nucleic acid strands relative to target nucleic acid strands results in extension of primers bound to target nucleic acid strands relative to wild-type nucleic acid strands, and
(e) extending the primers to enrich the target sequences.

In some embodiments, a method using simple PCR further comprises repeating at least once steps (c)-(e).

Any of the PCR or COLD-PCR techniques that utilize the generic probes as disclosed herein to enrich target nucleic acid comprising a microsatellite having a deletion relative to wild-type nucleic acid comprising a corresponding microsatellite with no deletion can be preceded by any of the NaME methods disclosed herein. When any of the NaME methods disclosed herein precedes any of the PCR methods disclosed herein, the DSN is inactivated prior to the PCR method.

Any of the PCR or COLD-PCR techniques that utilize the generic probes as disclosed herein to enrich target nucleic acid comprising a microsatellite having a deletion relative to wild-type nucleic acid comprising a corresponding microsatellite with no deletion can be follow by any of the NaME methods disclosed herein.

Genomic DNA may be fragmented before ligating to adaptors.

### Non-enzymatic enrichment of A:T microsatellites at multiple genomic targets using COLD-PCR

Also contemplated herein is a simple approach to enrich the A:T-rich genomic fragments or sequences over G:C rich fragments or sequences (including polyA/T with deletions and WT microsatellites without deletions), so that during sequencing, most of the sequencing reads concentrate or focus on the targets that contain the clinically useful information, i.e. the AT-rich sequences. In some embodiments, this approach is called 'target enrichment', as opposed to `deletion enrichment' which refers to enrichment of altered microsatellites relative to their WT alleles.

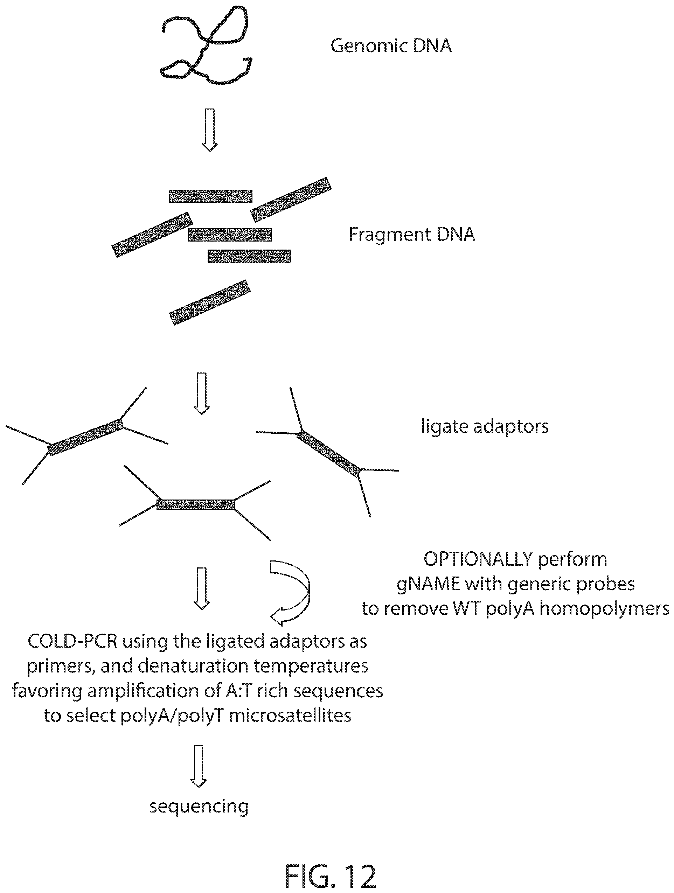

FIG. 12 demonstrates and example of target enrichment of the A:T-rich portion of the genome, which involves PCR amplification at lower denaturation temperature, COLD-PCR. COLD-PCR and its derivatives, e.g., Temperature-Tolerant COLD-PCR are disclosed in the following patent application: US 2014/0051087, US 2016/0186237, US20180282798, US8455190, US20160186237.

Following ligation of adaptors to the genomic fragments to be interrogated (e.g., circulating DNA from a blood sample; or genomic DNA from a tumor biopsy), a series of COLD-PCR cycles may be applied, where the denaturation temperature at each cycle is not high enough to denature fragments of high melting temperature Tm (e.g., high GC fragments), but it is adequate for denaturation of low Tm fragments like polynucleotide repeats that include polyA and polyT. For example, the denaturation temperature can be set to 70°C, or 75°C instead of say 95°C for denaturation during PCR.

Accordingly, also contemplated herein is a method of enriching A:T containing microsatellites in a sample of genomic DNA, the method comprising:
(a) providing a nucleic acid sample comprising at least a first double-stranded nucleic acid containing an A:T-rich microsatellite and at least a first double-stranded nucleic acid containing a G:C rich microsatellite, wherein each end of each strand of the double-stranded nucleic acids are ligated to an adaptor;
   providing a pair of nucleic acid primers that are complementary to the adaptors and under amplification conditions can amplify the adapter ligated double-stranded nucleic acids;
(b) forming a reaction mixture containing the nucleic acid sample and the pair of nucleic acid primers;
(c) subjecting the reaction mixture to a first critical denaturation temperature (Tc) to permit preferential denaturation of nucleic acids containing an A:T-rich microsatellite relative to nucleic acids containing a G:C rich microsatellite;
(f) reducing the temperature of the reaction mixture in the presence of pairs of nucleic acid primers and permitting the primers to anneal to the adaptors, and
(g) extending the primers to enrich the target sequences.

Any of the PCR or COLD-PCR techniques that utilize the generic probes as disclosed herein to enrich A:T-rich sequencing containing target nucleic acid can be preceded by any of the NaME methods disclosed herein. When any of the NaME methods disclosed herein precedes any of the PCR methods disclosed herein, the DSN is inactivated prior to the PCR method.

Any of the PCR or COLD-PCR techniques that utilize the generic probes as disclosed herein to enrich A:T-rich sequencing containing target nucleic acid can be follow by any of the NaME methods disclosed herein.

Genomic DNA may be fragmented before ligating to adaptors.

### Universal microsatellite target-enrichment using restriction endonucleases

It is further contemplated that microsatellite target enrichment may be accomplished by using one or more restriction endonucleases that are specific for GC-rich sequences. These would be anticipated to leave A:T-rich regions intact, such as polyA/T repeat mononucleotides, or AT dinucleotide repeats. Additionally, AC, AG dinucleotide repeats, or CAG trinucleotide repeats would also remain intact. On the other hand, other sequences as well as most of the flanking sequences around microsatellites would be digested heavily.

For example, by using CviPII enzyme (Chan et al., Nucleic Acids Res, 32, 6187-6199) which cuts at CCA and CCG (and to a lesser extent at CCT) the microsatellite BAT 26 shown on Figure 12A remains intact. In contrast, the remaining flanking sequences as well as all GC-rich part of the genome are digested in small fragments and can be removed by size-selection filtration, or by additional digestion with another -CG-targeting enzyme.

Next, by ligating adaptors to the undigested microsatellite sequences, a ligation-mediated PCR using standard PCR conditions (or COLD-PCR with reduced denaturation temp) can form a DNA library that can be sequenced. Accordingly, sequencing can be focused almost exclusively at microsatellites throughout the genome, thus saving cost, time and effort.

CviPII can be made to cut only at CCA sequences, and not at CCG. Because the enzyme does not cut CG-methylated DNA (CpG sites), the DNA that is to be screened can first be fully methylated by treatment with DNA methyltransferases or other enzymes that methylate the G within CpG sequences. Then subsequent treatment with CviPII can be regulated to cut only CCA. In this way the generation of fragments from enzymatic treatment can be adapted as needed, to perform either very frequent cutting or modestly frequent cutting.

While both approaches to enrich A:T-rich regions as disclosed herein (i.e., using COLD-PCR and using GC-enzyme cutter) are able to generate target enrichment at polyA or polyT mononucleotide repeats, the COLD-PCR methods have the advantage of simplicity (no enzymatic cutting), while enzymatic cutting methods have the advantage that they can enrich more complex microsatellites, like dinucleotides (CA)n or trinucleotides (GAC)n more easily, or also higher order microsatellites, since the approach does not depend on the Tm of the microsatellite. Thus even a high Tm microsatellite like (GAC)n will also be preferentially enriched easily.

### Nucleic acid samples

The method disclosed herein can be used to enhance identification of microsatellite deletions in tumors, circulating DNA or in other liquid biopsies obtained from a subject (e.g., a human subject suffering from or is suspected to suffer from cancer), for detection of minimal residual disease, tumor mutational burden, or to monitor tumor dynamics. Alternatively, the method disclosed herein can be used to screen apparently healthy individuals for signs of early disease (e.g., cancer) by periodic testing of biological samples (e.g., blood, urine, buccal swap, or tissue biopsy).

In some embodiments, genomic DNA is cell-free circulating DNA. In some embodiments, genomic DNA is obtained from a biological sample. In some embodiments, a biological sample is serum, plasma, blood, urine, solid tissue (e.g., a tumor), feces, skin, hair, a buccal swab, or a pulmonary brushing.

### Use of methods as a marker for personalized tumor

Small deletions or insertions (commonly 1-30bp in size) at microsatellites take place even in the absence of mismatch repair deficiency and microsatellite instability, but at a lower level than in repair-deficient cells. Such indels are clonally expanded in tumors due to tumor cell proliferation. The specific indel distribution in a tumor genome can serve as a signature for that tumor. The signature can then be traced in the blood as a tumor biomarker, thereby serving to identify minimal residual disease during or after cancer therapy.

### EXAMPLES

### Reference example 1: Experimental validation of g-NaME

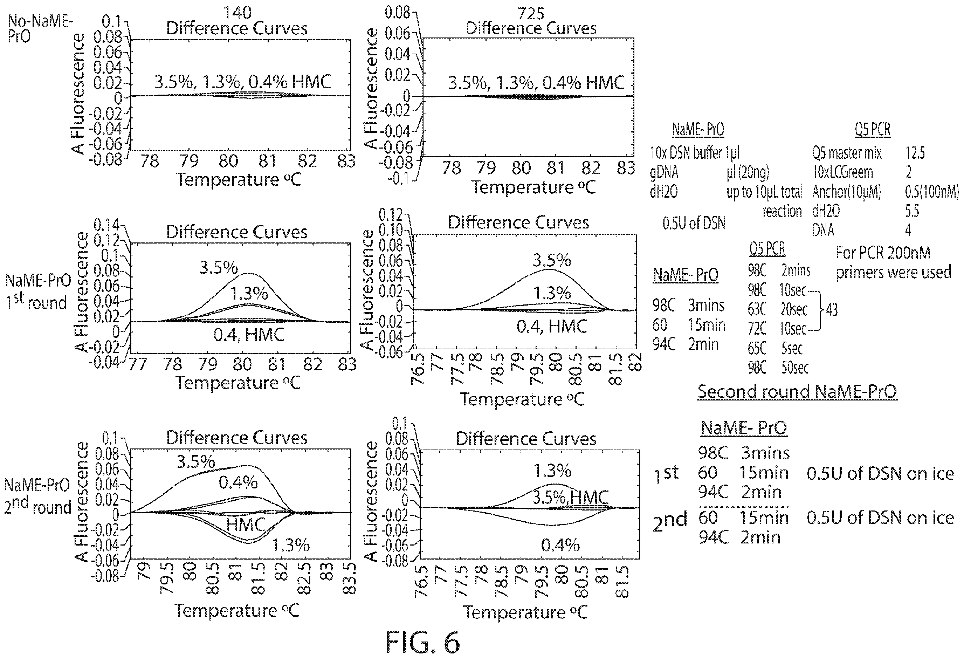

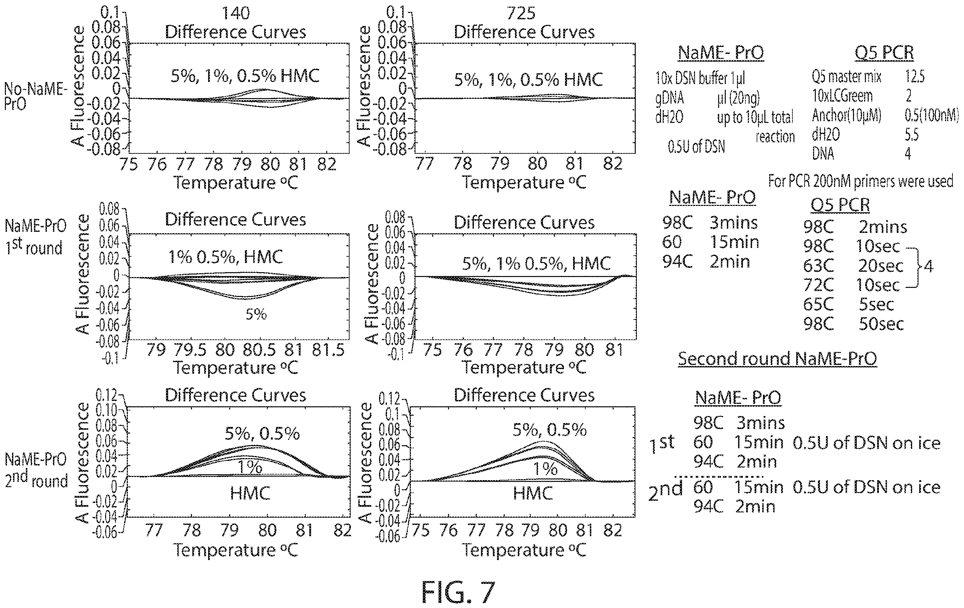

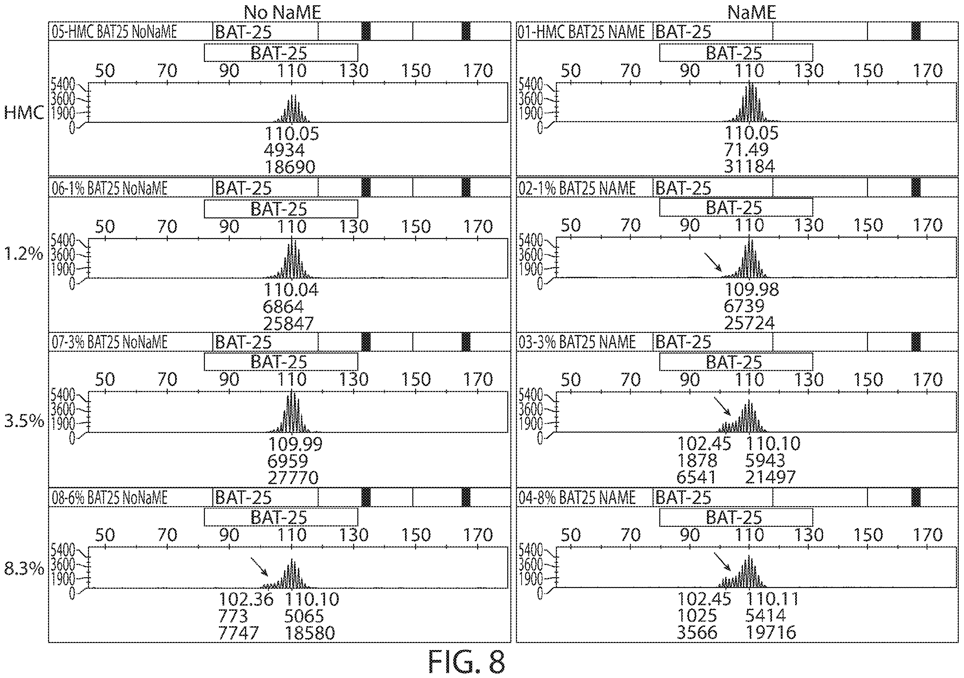

FIGs. 6 and 7 demonstrate experimental validation for the self- NaME embodiment as shown in FIG. 4. Two randomly selected poly-A15 microsatellites (#725 and #140) were tested using mixtures of DNA from deletion-containing cell lines and wild type DNA, and then tested following application of the no-probe self- NaME protocol. PCR was then applied and the presence of deletion was tested via high resolution melting (HRM). The deletion can be detected after application of self- NaME, while when NO- NaME was run in parallel as a control the deletion cannot be detected. FIG. 8 demonstrates similar results by using capillary electrophoresis as the endpoint detection. Since the microsatellites tested were randomly chosen, it is likely that the enrichment of deletions is taking place over the entire population of poly-A15 microsatellites in the genome.

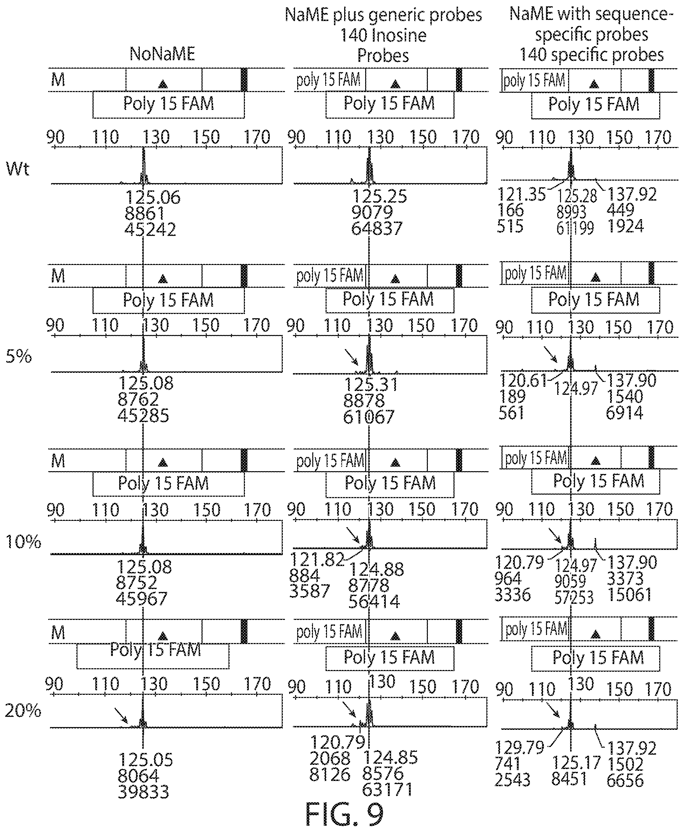

FIG. 9 demonstrates similar conclusions as FIG. 8, but by using generic probes with inosines at the two ends, directed against both the sense and the antisense strands containing the randomly-chosen microsatellite #140.

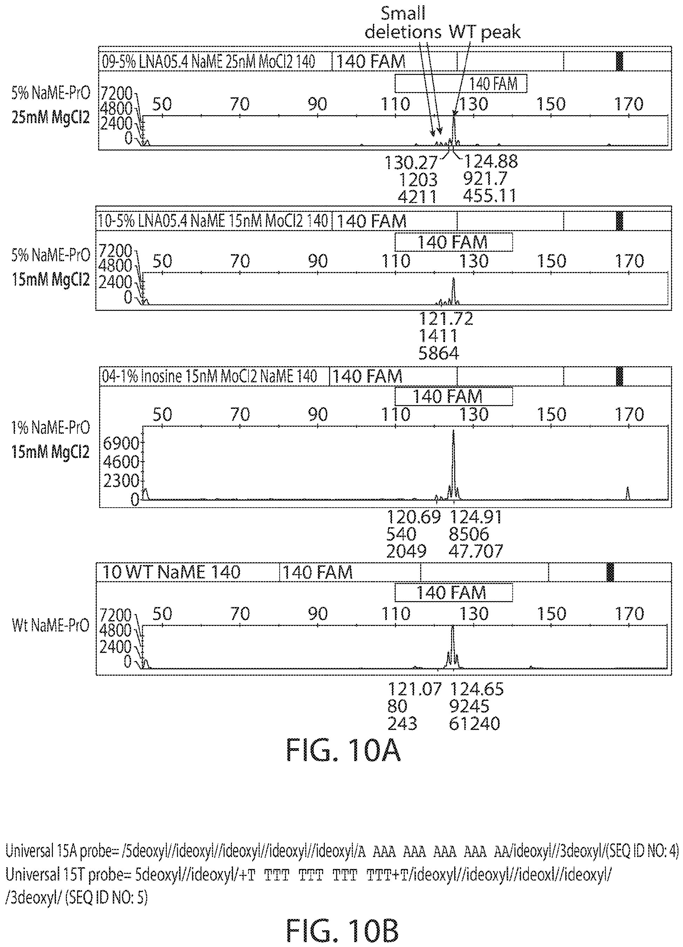

FIG. 10A demonstrates that the compositions and materials shown in FIG. 9 show enhanced enrichment of the same randomly-chosen microsatellite #140 when the Mg++ concentration during NaME-PrO using probes as shown in FIG. 10B is increased to 15mM or 25mM instead of the standard 5mM.

### Reference example 2: Enriching A:T-rich regions using endonucleases

FIG. 13A shows a method of using the frequent cutting enzyme CviPII enzyme which cuts at CCA and CCG (and to a lesser extent at CCT) the microsatellite BAT 26. In contrast, the remaining flanking sequences as well as all GC-rich part of the genome are digested in small fragments and can be removed by size-selection filtration, or by additional digestion with another -CG-targeting enzyme.

Next, by ligating adaptors to the undigested microsatellite sequences, a ligation-mediated PCR using standard PCR conditions (not necessarily COLD-PCR with reduced denaturation temp) can form a DNA library that can be sequenced. Accordingly, sequencing can be focused almost exclusively at microsatellites throughout the genome, thus saving cost, time and effort.

FIG. 13B shows use of the CviPII to cut only at CCA sequences, and not at CCG. Because the enzyme does not cut CG-methylated DNA (CpG sites), the DNA that is to be screened can first be fully methylated by treatment with DNA methyltransferases or other enzymes that methylate the G within CpG sequences. Then the subsequent treatment with CviPII can be regulated to cut only CCA. In this way the generation of fragments from enzymatic treatment can be adapted as needed, to perform either very frequent cutting or modestly frequent cutting.

### REFERENCES

1. Thomas, R.K., Baker, A.C., Debiasi, R.M., Winckler, W., Laframboise, T., Lin, W.M., Wang, M., Feng, W., Zander, T., Macconnaill, L.E. et al. (2007) High-throughput oncogene mutation profiling in human cancer. Nat Genet, 39, 347-351.
2. Chou, L.S., Lyon, E. and Wittwer, C.T. (2005) A comparison of high-resolution melting analysis with denaturing high-performance liquid chromatography for mutation scanning: cystic fibrosis transmembrane conductance regulator gene as a model. Am J Clin Pathol, 124, 330-338.
3. Thomas, R.K., Nickerson, E., Simons, J.F., Janne, P.A., Tengs, T., Yuza, Y., Garraway, L.A., Laframboise, T., Lee, J.C., Shah, K. et al. (2006) Sensitive mutation detection in heterogeneous cancer specimens by massively parallel picoliter reactor sequencing. Nat Med, 12, 852-855.
4. Paez, J.G., Janne, P.A., Lee, J.C., Tracy, S., Greulich, H., Gabriel, S., Herman, P., Kaye, F.J., Lindeman, N., Boggon, T.J. et al. (2004) EGFR Mutations in Lung Cancer: Correlation with Clinical Response to Gefitinib Therapy. Science, 304, 1497-1500.
5. Janne, P.A., Borras, A.M., Kuang, Y., Rogers, A.M., Joshi, V.A., Liyanage, H., Lindeman, N., Lee, J.C., Halmos, B., Maher, E.A. et al. (2006) A rapid and sensitive enzymatic method for epidermal growth factor receptor mutation screening. Clin Cancer Res, 12, 751-758.
6. Engelman, J.A., Mukohara, T., Zejnullahu, K., Lifshits, E., Borras, A.M., Gale, C.M., Naumov, G.N., Yeap, B.Y., Jarrell, E., Sun, J. et al. (2006) Allelic dilution obscures detection of a biologically significant resistance mutation in EGFR -amplified lung cancer. J Clin Invest.
7. Diehl, F., Li, M., Dressman, D., He, Y., Shen, D., Szabo, S., Diaz, L.A., Jr., Goodman, S.N., David, K.A., Juhl, H. et al. (2005) Detection and quantification of mutations in the plasma of patients with colorectal tumors. Proc Natl Acad Sci USA, 102, 16368-16373.
8. Kimura, T., Holland, W.S., Kawaguchi, T., Williamson, S.K., Chansky, K., Crowley, J.J., Doroshow, J.H., Lenz, H.J., Gandara, D.R. and Gumerlock, P.H. (2004) Mutant DNA in plasma of lung cancer patients: potential for monitoring response to therapy. Ann N Y Acad Sci, 1022, 55-60.
9. Bacher, J.W., Sievers, C.K., Albrecht, D.M., Grimes, I.C., Weiss, J.M., Matkowskyj, K.A., Agni, R.M., Vyazunova, I., Clipson, L., Storts, D.R. et al. (2015) Improved Detection of Microsatellite Instability in Early Colorectal Lesions. PLoS One, 10, e0132727.
10. Beggs, A.D., Domingo, E., Abulafi, M., Hodgson, S.V. and Tomlinson, I.P. (2013) A study of genomic instability in early preneoplastic colonic lesions. Oncogene, 32, 5333-5337.
11. Shaw, J.A., Smith, B.M., Walsh, T., Johnson, S., Primrose, L., Slade, M.J., Walker, R.A. and Coombes, R.C. (2000) Microsatellite alterations plasma DNA of primary breast cancer patients. Clin Cancer Res, 6, 1119-1124.
12. Buhard, O., Lagrange, A., Guilloux, A., Colas, C., Chouchène, M., Wanherdrick, K., Coulet, F., Guillerm, E., Dorard, C., Marisa, L. et al. (2016) HSP110 T17 simplifies and improves the microsatellite instability testing in patients with colorectal cancer. J Med Genet, 53, 377-384.
13. Li, J., Wang, L., Mamon, H., Kulke, M.H., Berbeco, R. and Makrigiorgos, G.M. (2008) Replacing PCR with COLD-PCR enriches variant DNA sequences and redefines the sensitivity of genetic testing. Nat Med, 14, 579-584.
14. Milbury, C.A., Correll, M., Quackenbush, J., Rubio, R. and Makrigiorgos, G.M. (2012) COLD-PCR enrichment of rare cancer mutations prior to targeted amplicon resequencing. Clin Chem, 58, 580-589.
15. How Kit, A., Mazaleyrat, N., Daunay, A., Nielsen, H.M., Terris, B. and Tost, J. (2013) Sensitive detection of KRAS mutations using enhanced-ice-COLD-PCR mutation enrichment and direct sequence identification. Hum Mutat, 34, 1568-1580.
16. How-Kit, A., Daunay, A., Buhard, O., Meiller, C., Sahbatou, M., Collura, A., Duval, A. and Deleuze, J.F. (2017) Major improvement in the detection of microsatellite instability in colorectal cancer using HSP110 T17 E-ice-COLD-PCR. Hum Mutat.
17. Ellegren, H. (2004) Microsatellites: simple sequences with complex evolution. Nat Rev Genet, 5, 435-445.
18. Milbury, C.A., Li, J. and Makrigiorgos, G.M. (2009) PCR-based methods for the enrichment of minority alleles and mutations. Clin Chem, 55, 632-640.
19. Janavicius, R., Matiukaite, D., Jakubauskas, A. and Griskevicius, L. (2010) Microsatellite instability detection by high-resolution melting analysis. Clin Chem, 56, 1750-1757.
20. Gan, C., Love, C., Beshay, V., Macrae, F., Fox, S., Waring, P. and Taylor, G. (2015) Applicability of next generation sequencing technology in microsatellite instability testing. Genes (Basel), 6, 46-59.
21. Maruvka, Y.E., Mouw, K.W., Karlic, R., Parasuraman, P., Kamburov, A., Polak, P., Haradhvala, N.J., Hess, J.M., Rheinbay, E., Brody, Y. et al. (2017) Analysis of somatic microsatellite indels identifies driver events in human tumors. Nat Biotechnol, 35, 951-959.
22. Ladas, I., Yu, F., Leong, K.W., Fitarelli-Kiehl, M., Song, C., Ashtaputre, R., Kulke, M., Mamon, H. and Makrigiorgos, G.M. (2018) Enhanced detection of microsatellite instability using pre-PCR elimination of wild-type DNA homo-polymers in tissue and liquid biopsies. Nucleic Acids Res, 46, e74.
23. Chan, S.H., Zhu, Z., Van Etten, J.L. and Xu, S.Y. (2004) Cloning of CviPII nicking and modification system from chlorella virus NYs-1 and application of Nt.CviPII in random DNA amplification. Nucleic Acids Res, 32, 6187-6199.

It is to be understood that the foregoing embodiments are presented by way of example only.

All definitions, as defined and used herein, should be understood to control over dictionary definitions, definitions in documents referenced herein, and/or ordinary meanings of the defined terms.

The indefinite articles "a" and "an," as used herein in the specification and in the claims, unless clearly indicated to the contrary, should be understood to mean "at least one."

The phrase "and/or" should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

The phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

It should also be understood that, unless clearly indicated to the contrary, in any methods claimed herein that include more than one step or act, the order of the steps or acts of the method is not necessarily limited to the order in which the steps or acts of the method are recited.

All transitional phrases such as "comprising," "including," "carrying," "having," "containing," "involving," "holding," "composed of," and the like are to be understood to be open-ended, i.e., to mean including but not limited to. Only the transitional phrases "consisting of" and "consisting essentially of" shall be closed or semi-closed transitional phrases, respectively, as set forth in the United States Patent Office Manual of Patent Examining Procedures, Section 2111.03. It should be appreciated that embodiments described in this document using an open-ended transitional phrase (e.g., "comprising") are also contemplated, in alternative embodiments, as "consisting of" and "consisting essentially of" the feature described by the open-ended transitional phrase. For example, if the disclosure describes "a composition comprising A and B", the disclosure also contemplates the alternative embodiments "a composition consisting of A and B" and "a composition consisting essentially of A and B".

## Claims

1. A method for enriching deletion-containing microsatellite targets in a sample of genomic DNA, the method comprising:
providing a nucleic acid sample comprising at least a first double-stranded wild-type nucleic acid containing a microsatellite and at least a first double-stranded target nucleic acid suspected of containing a microsatellite corresponding to the wild-type microsatellite with at least one deletion relative to the wild-type microsatellite;
adding to the nucleic acid sample a pair of oligonucleotide probes comprising a first probe and a second probe, wherein the first probe comprises a region that is complementary to the microsatellite sequence in the wild-type nucleic acid top strand and the second probe comprises a region that is complementary to the microsatellite sequence in the wild-type nucleic acid bottom strand; wherein the length of the region that is complementary to the microsatellite sequences is at least 5 nucleotides;
adding to the nucleic acid sample a double strand specific nuclease (DSN);
subjecting the nucleic acid sample to a first temperature that destabilizes or denatures the at least first double-stranded wildtype and the at least first target mutant nucleic acids;
subjecting the nucleic acid sample to a second temperature that allows preferential formation of complementary wild-type nucleic acid-probe duplexes relative to partially complementary target nucleic acid-probe duplexes; and
subjecting the nucleic acid sample to a third temperature that allows the DSN to preferentially cleave the complementary wild-type nucleic acid-probe duplexes relative to partially complementary target nucleic acid-probe duplexes;
wherein the DSN is added:
(i) after subjecting the nucleic acid sample to the second temperature, or
(ii) before subjecting the nucleic acid sample to the first temperature, and wherein the method further comprises adding to the nucleic acid sample an organic solvent that lowers the melting temperature of the wild-type and target nucleic acids to inhibit inactivation of the DSN when the nucleic acid sample is subjected to the first temperature.

2. The method of claim 1, wherein the first and second oligonucleotide probes each comprise at least one locked nucleic acid (LNA), peptide nucleic acid (PNA), or xeno nucleic acid (XNA).

3. The method of claim 2, wherein the first and second oligonucleotide probes each comprise 2 to 5 LNAs, PNAs, or XNAs, wherein the distance between the innermost LNAs, PNAs, or XNAs is greater than 10 nucleotides.

4. The method of any one of the preceding claims, wherein the region of the first and second probes that is complementary to the microsatellite sequence in the top and bottom strands of the wild-type nucleic acid is 5-100 nucleotides long.

5. The method of claim 4, wherein the first and second probes comprise 1-20 inosines that flank the region that is complementary to the microsatellite sequence on each side.

6. The method of claim 5, wherein the number of inosines in the 5' end of the first probe is not equal to the number of inosines in the 3' end of the second probe, and the number of inosines in the 3' end of the first probe is not equal to the number of inosines in the 5' end of the second probe.

7. The method of any one of the preceding claims, wherein the first and second probes are 5 to 150 nucleotides long.

8. The method of any one of the preceding claims, further comprising adding an Mg²⁺ salt to the nucleic acid sample so that the concentration of Mg²⁺ in the nucleic acid sample is between 15-25 mM when the nucleic acid sample is subjected to the second temperature and/or third temperature.

9. The method of any one of the preceding claims, wherein the microsatellite is a mono-nucleotide repeat, di-nucleotide repeat, or tri-nucleotide repeat.

10. The method of any one of the preceding claims, wherein the first and second probes are in molar excess of 100-fold to 1 billion-fold compared to wild-type and target nucleic acids.

## Patentansprüche

1. Verfahren zur Anreicherung von Deletionen enthaltenden Mikrosatellitenzielen in einer Probe genomischer DNA, wobei das Verfahren Folgendes umfasst:
Bereitstellen einer Nukleinsäureprobe, die zumindest eine erste doppelsträngige Wildtyp-Nukleinsäure, die einen Mikrosatelliten enthält, und zumindest eine erste doppelsträngige ZielNukleinsäure, von der angenommen wird, dass sie einen Mikrosatelliten enthält, der dem Wildtyp-Mikrosatelliten entspricht, mit zumindest einer Deletion relativ zu dem Wildtyp-Mikrosatelliten umfasst;
Hinzufügen, zu der Nukleinsäureprobe, eines Paars Oligonukleotidsonden, das eine erste Sonde und eine zweite Sonde umfasst, wobei die erste Sonde einen Bereich umfasst, der komplementär zu der Mikrosatellitensequenz in dem oberen Strang der Wildtyp-Nukleinsäure ist und die zweite Sonde einen Bereich umfasst, der komplementär zu der Mikrosatellitensequenz in dem unteren Strang der Wildtyp-Nukleinsäure ist; wobei die Länge des Bereichs, der komplementär zu den Mikrosatellitensequenzen ist, zumindest 5 Nukleotide ist;
Hinzufügen einer doppelstrangspezifischen Nuklease (DSN) zu der Nukleinsäureprobe;
Aussetzen der Nukleinsäureprobe einer ersten Temperatur, welche die zumindest erste doppelsträngige Wildtyp- und die zumindest ersten Zielmutantennukleinsäuren destabilisiert oder denaturiert;
Aussetzen der Nukleinsäureprobe einer zweiten Temperatur, die bevorzugte Bildung von komplementären Wildtyp-Nukleinsäure-Sonden-Duplexen relativ zu teilweise komplementären Zielnukleinsäure-Sonden-Duplexen ermöglicht; und
Aussetzen der Nukleinsäureprobe einer dritten Temperatur, die der DSN ermöglicht, die komplementären Wildtyp-Nukleinsäure-Sonden-Duplexe relativ zu teilweise komplementären Zielnukleinsäure-Sonden-Duplexen bevorzugt zu spalten;
wobei die DSN hinzugefügt wird:
(i) nach dem Aussetzen der Nukleinsäureprobe der zweiten Temperatur, oder
(ii) vor dem Aussetzen der Nukleinsäureprobe der ersten Temperatur, und wobei das Verfahren ferner Hinzufügen eines organischen Lösungsmittels zu der Nukleinsäureprobe umfasst, das die Schmelztemperatur der Wildtyp- und Zielnukleinsäuren senkt, um Inaktivierung der DSN zu verhindern, wenn die Nukleinsäureprobe der ersten Temperatur ausgesetzt wird.

2. Verfahren nach Anspruch 1, wobei die erste und die zweite Oligonukleotidsonde jeweils zumindest eine verbrückte Nukleinsäure (LNA), Peptidnukleinsäure (PNA) oder Xenonukleinsäure (XNA) umfassen.

3. Verfahren nach Anspruch 2, wobei die erste und die zweite Oligonukleotidsonde jeweils 2 bis 5 LNAs, PNAs oder XNAs umfassen, wobei der Abstand zwischen den innersten LNAs, PNAs oder XNAs größer als 10 Nukleotide ist.

4. Verfahren nach einem der vorhergehenden Ansprüche, wobei der Bereich der ersten und der zweiten Sonde, der komplementär zu der Mikrosatellitensequenz in dem oberen und unteren Strang der Wildtyp-Nukleinsäure ist, 5-100 Nukleotide lang ist.

5. Verfahren nach Anspruch 4, wobei die erste und die zweite Sonde 1-20 Inosine umfassen, die den Bereich flankieren, der auf jeder Seite komplementär zu der Mikrosatellitensequenz ist.

6. Verfahren nach Anspruch 5, wobei die Anzahl an Inosinen an dem 5'-Ende der ersten Sonde nicht gleich der Anzahl an Inosinen an dem 3'-Ende der zweiten Sonde ist und die Anzahl an Inosinen an dem 3'-Ende der ersten Sonde nicht gleich der Anzahl an Inosinen an dem 5'-Ende der zweiten Sonde ist.

7. Verfahren nach einem der vorhergehenden Ansprüche, wobei die erste und die zweite Sonde 5 bis 150 Nukleotide lang sind.

8. Verfahren nach einem der vorhergehenden Ansprüche, ferner umfassend Hinzufügen eines Mg²⁺-Salzes zu der Nukleinsäureprobe, sodass die Konzentration von Mg²⁺ in der Nukleinsäureprobe zwischen 15-25 mM ist, wenn die Nukleinsäureprobe der zweiten Temperatur und/oder der dritten Temperatur ausgesetzt wird.

9. Verfahren nach einem der vorhergehenden Ansprüche, wobei der Mikrosatellit eine Mononukleotidwiederholung, Dinukleotidwiederholung oder Trinukleotidwiederholung ist.

10. Verfahren nach einem der vorhergehenden Ansprüche, wobei die erste und die zweite Sonde in molarem Überschuss von 100-fach bis 1 Milliarde-fach verglichen mit Wildtyp- und Zielnukleinsäuren sind.

## Revendications

1. Méthode permettant d'enrichir des cibles microsatellites contenant des délétions dans un échantillon d'ADN génomique, la méthode comprenant :
la fourniture d'un échantillon d'acide nucléique comprenant au moins un premier acide nucléique de type sauvage double brin contenant un microsatellite et au moins un premier acide nucléique cible double brin soupçonné de contenir un microsatellite correspondant au microsatellite de type sauvage avec au moins une délétion par rapport au microsatellite de type sauvage ;
l'ajout à l'échantillon d'acide nucléique d'une paire de sondes oligonucléotidiques comprenant une première sonde et une seconde sonde, ladite première sonde comprenant une région qui est complémentaire à la séquence de microsatellite dans le brin supérieur d'acide nucléique de type sauvage et ladite seconde sonde comprenant une région qui est complémentaire à la séquence de microsatellite dans le brin inférieur d'acide nucléique de type sauvage ; la longueur de la région qui est complémentaire aux séquences de microsatellite étant d'au moins 5 nucléotides ;
l'ajout à l'échantillon d'acide nucléique d'une nucléase spécifique aux doubles brins (DSN) ;
la soumission de l'échantillon d'acide nucléique à une première température qui déstabilise ou dénature ledit au moins un premier acide nucléique de type sauvage double brin et ledit au moins un premier acide nucléique mutant cible ;
la soumission de l'échantillon d'acide nucléique à une deuxième température qui permet la formation préférentielle de duplexes acide nucléique de type sauvage/sonde complémentaires par rapport aux duplexes acide nucléique cible/sonde partiellement complémentaires ; et
la soumission de l'échantillon d'acide nucléique à une troisième température qui permet à la DSN de cliver préférentiellement les duplexes acide nucléique de type sauvage/sonde complémentaires par rapport aux duplexes acide nucléique cible/sonde partiellement complémentaires ;
ladite DSN étant ajoutée :
(i) après la soumission de l'échantillon d'acide nucléique à la deuxième température, ou
(ii) avant la soumission de l'échantillon d'acide nucléique à la première température, et ladite méthode comprenant en outre l'ajout à l'échantillon d'acide nucléique d'un solvant organique qui abaisse la température de fusion des acides nucléiques cible et de type sauvage pour inhiber l'inactivation de la DSN lorsque l'échantillon d'acide nucléique est soumis à la première température.

2. Méthode selon la revendication 1, lesdites première et seconde sondes oligonucléotidiques comprenant chacune au moins un acide nucléique verrouillé (LNA), un acide nucléique peptidique (PNA) ou un acide xénonucléique (XNA).

3. Méthode selon la revendication 2, lesdites première et seconde sondes oligonucléotidiques comprenant chacune 2 à 5 LNA, PNA ou XNA, la distance entre les LNA, PNA ou XNA les plus internes étant supérieure à 10 nucléotides.

4. Méthode selon l'une quelconque des revendications précédentes, ladite région des première et seconde sondes qui est complémentaire à la séquence de microsatellite dans les brins supérieur et inférieur de l'acide nucléique de type sauvage faisant 5 à 100 nucléotides de long.

5. Méthode selon la revendication 4, lesdites première et seconde sondes oligonucléotidiques comprenant 1 à 20 inosines qui flanquent la région qui est complémentaire à la séquence de microsatellite de chaque côté.

6. Méthode selon la revendication 5, le nombre d'inosines dans l'extrémité 5' de la première sonde n'étant pas égal au nombre d'inosines dans l'extrémité 3' de la seconde sonde, et le nombre d'inosines dans l'extrémité 3' de la première sonde n'étant pas égal au nombre d'inosines dans l'extrémité 5' de la seconde sonde.

7. Méthode selon l'une quelconque des revendications précédentes, lesdites première et seconde sondes oligonucléotidiques faisant 5 à 150 nucléotides de long.

8. Méthode selon l'une quelconque des revendications précédentes, comprenant en outre l'ajout d'un sel de Mg²⁺ à l'échantillon d'acide nucléique de sorte que la concentration de Mg²⁺ dans l'échantillon d'acide nucléique soit comprise entre 15 à 25 mM lorsque l'échantillon d'acide nucléique est soumis à la deuxième température et/ou à la troisième température.

9. Méthode selon l'une quelconque des revendications précédentes, ledit microsatellite étant une répétition mono-nucléotidique, une répétition di-nucléotidique ou une répétition trinucléotidique.

10. Méthode selon l'une quelconque des revendications précédentes, lesdites première et seconde sondes oligonucléotidiques se trouvant dans un excès molaire d'un facteur 100 à un facteur de 1 milliard par rapport aux acides nucléiques cible et de type sauvage.
